# EUROPEAN PATENT APPLICATION

(11) **EP 3 835 423 A1**
(43) Date of publication of application: **16.06.2021**
(21) Application number: 19846502.3
(22) Date of filing: 08.08.2019
(51) Int. Cl.: C12N 15/113, C12N 5/10, C12N 15/85, C12P 21/02

(54) **PROMOTOR FOR HSPA8 GENE**

(30) Priority: 09.08.2018 JP 2018149854
(71) Applicant: Daiichi Sankyo Company, Limited, Tokyo 103-8426 (JP)
(72) Inventor: MASUDA Kenji, Tokyo 103-8426 (JP); NAKAZAWA Yuto, Tokyo 103-8426 (JP); WATANABE Kazuhiko, Tokyo 103-8426 (JP); NISHIO Maui, Tokyo 103-8426 (JP); OKUMURA Takeshi, Tokyo 103-8426 (JP); NONAKA Koichi, Tokyo 103-8426 (JP)
(74) Representative: Arends, William Gerrit
(86) International application number: PCT/JP2019/031295
(87) International publication number: WO 2020/032153

(57) **Abstract**

The present invention provides an approach to enhancing the production of a foreign protein serving as a protein-based pharmaceutical product in host cells such as cultured cells derived from a mammal. The present invention provides transfected cells having a novel Hspa8 gene promoter, and a method for secreting and producing a foreign protein at high levels using the transfected cells.

## Description

### Technical Field

The present invention relates to transfected mammalian cells with enhanced transcriptional activity in relation to a foreign protein, which is obtained by using a foreign gene expression vector having a Hspa8 gene promoter, and a method for producing the foreign protein using the same.

### Background Art

The development of gene recombination techniques has rapidly expanded the market of protein-based pharmaceutical products such as therapeutic proteins and antibody drugs. Among them, antibody drugs do not cause adverse immune responses when administered to the human body and are under active development because of their high specificity.

Examples of hosts to produce the protein-based pharmaceutical products typified by antibody drugs can include microorganisms, yeasts, insects, animal and plant cells, and transgenic animals and plants. Posttranslational modification such as folding or sugar chain modification is essential for the physiological activity or antigenicity of the protein-based pharmaceutical products. Therefore, microorganisms which cannot perform complicated posttranslational modification, or plants which differ significantly in sugar chain structure from humans are not suitable as hosts. Cultured mammalian cells, such as CHO (Chinese hamster ovary) cells, are currently mainstream due to their having a sugar chain structure similar to that of humans and permitting posttranslational modification, and further in consideration of safety.

Use of cultured mammalian cells as a host presents problems such as low growth rates, low productivity and high cost as compared with microorganisms or the like (Non Patent Literature 1). Furthermore, clinical utilization of protein-based pharmaceutical products requires administering the pharmaceutical products at large doses. Therefore, a lack of sufficient production capacity thereof has been a global issue. In the case of producing a protein-based pharmaceutical product in a cultured mammalian cell expression system, reduction in production cost has been attempted by making improvements to each production step, because the production cost is higher than that of synthetic low-molecular weight pharmaceutical products. However, increasing the amount of protein produced in the cultured mammalian cell expression system is also a promising method for reduction in production cost (Non Patent Literature 2 and 3). Accordingly, in order to increase the productivity of foreign genes in cultured mammalian cells, many approaches such as promoters, enhancers, drug selection markers, gene amplification and culture engineering approaches have been practiced so far through trial and error. In the case of using CHO cells as host cells, a human cytomegalovirus major immediate early promoter (hereinafter, referred to as a CMV promoter) derived from a virus is generally used for the expression of foreign genes, i.e., the production of protein-based pharmaceutical products (Non Patent Literature 4, 5 and 6). It is also known that a polynucleotide upstream of the transcription start site of the gene (promoter region) of elongation factor-1 alpha (EF-1α) (Patent Literature 1 and Non Patent Literature 7) or a human ribosomal protein RPL32 or RPS11 can be used alone or in combination with an additional heterologous promoter in protein expression in CHO cells (Non Patent Literature 8 and Patent Literature 2 and 3). The heat-shock protein A5 (Hspa5/GRP78) gene promoter is known to improve the productivity of a foreign protein in mammalian cells (Patent Literature 4).

### Citation List

### Patent Literature

Patent Literature 1: Japanese Patent No. 3051411
Patent Literature 2: WO2006/123097
Patent Literature 3: WO2013/080934
Patent Literature 4: WO2018/066492

### Non Patent Literature

Non Patent Literature 1: Florian M. Wurm., Nat. Biotechnol. 22 (11): 1393-1398, 2004
Non Patent Literature 2: Farid SS., J Chromatogr B Analyt Technol Biomed Life Sci. 848 (1): 8-18, 2007
Non Patent Literature 3: Werner RG. Economic aspects of commercial manufacture of biopharmaceuticals. J Biotechnol. 113 (1-3): 171-182, 2004
Non Patent Literature 4: Durocher Y et al., Curr Opin Biotechnol. 20 (6): 700-707, 2009
Non Patent Literature 5: Boshart M et al., Cell. 41 (2): 521-530, 1985
Non Patent Literature 6: Foecking MK et al., Gene. 45 (1): 101-105, 1986
Non Patent Literature 7: Deer JR. and Allison DS., Biotechnol. Prog. 20: 880-889, 2004
Non Patent Literature 8: Hoeksema F. et al., Biotechnology Research International, Volume 2011, Article ID 492875, 11 pages

### Summary of Invention

### Technical Problem

An object of the present invention is to provide a promoter having high foreign gene expression-enhancing activity in host cells such as cultured mammalian cells, and to provide an approach to enhancing the amount of a foreign protein, which serves as a protein-based pharmaceutical product, produced using the promoter. If a promoter is found which has promoter activity comparable to or higher than that of a human EF-1α promoter in CHO cells or the like, an approach to achieving the stable and high expression of a foreign gene in mammalian cells can be provided. Accordingly, an approach can be provided which contributes to increasing the amount of a protein-based pharmaceutical product produced in a cultured mammalian cell expression system, i.e., reduction in production cost.

### Solution to Problem

The present inventors have conducted intensive studies directed towards achieving the aforementioned object. As a result, the inventors have found that a polynucleotide approximately 2.9 kbp upstream of the start codon of a heat-shock protein A8 (Hspa8) gene has excellent promoter activity and is capable of markedly improving the productivity of a foreign protein to be expressed in cultured mammalian cells, thereby completing the present invention. Specifically, the present invention includes the following aspects of the invention.
(1) A polynucleotide consisting of the nucleotide sequence shown in SEQ ID NO: 1 or a partial sequence of the nucleotide sequence, the polynucleotide being a Chinese hamster derived Hspa8 gene promoter and comprising a polynucleotide consisting of the nucleotide sequence shown in SEQ ID NO: 6.
(2) The polynucleotide according to the above (1), which consists of the nucleotide sequence shown in SEQ ID NO: 1.
(3) The polynucleotide according to the above (1), which consists of the nucleotide sequence shown in SEQ ID NO: 5.
(4) The polynucleotide according to the above (1), which consists of the nucleotide sequence shown in SEQ ID NO: 6.
(5) A polynucleotide consisting of the nucleotide sequence shown in SEQ ID NO: 2 in the sequence listing, the polynucleotide being a human-derived Hspa8 gene promoter.
(6) A polynucleotide consisting of the nucleotide sequence shown in SEQ ID NO: 3 in the sequence listing, the polynucleotide being a mouse-derived Hspa8 gene promoter.
(7) A polynucleotide consisting of the nucleotide sequence shown in SEQ ID NO: 4 in the sequence listing, the polynucleotide being a rat-derived Hspa8 gene promoter.
(8) A polynucleotide consisting of a nucleotide sequence having 95% or higher identity to the nucleotide sequence according to any one of the above (1) to (7), the polynucleotide having promoter activity.
(9) A polynucleotide consisting of a nucleotide sequence having 99% or higher identity to the nucleotide sequence according to any one of the above (1) to (7), the polynucleotide having promoter activity.
(10) A polynucleotide that hybridizes under stringent conditions to a polynucleotide consisting of a nucleotide sequence complementary to the nucleotide sequence according to any one of the above (1) to (9), the polynucleotide having promoter activity.
(11) A foreign gene expression unit comprising the polynucleotide according to any one of the above (1) to (10) .
(12) The foreign gene expression unit according to the above (11), wherein the foreign gene is a gene encoding a multimeric protein.
(13) The foreign gene expression unit according to the above (11), wherein the foreign gene is a gene encoding a heteromultimeric protein.
(14) The foreign gene expression unit according to the above (11), wherein the foreign gene is a gene encoding an antibody or an antigen-binding fragment thereof.
(15) A foreign gene expression vector comprising the foreign gene expression unit according to any one of the above (11) to (14).
(16) A foreign gene expression vector comprising the foreign gene expression unit according to any one of the above (11) to (14) and any one or more polynucleotides selected from polynucleotides (a) to (e) of the following group A:
   group A
   (a) a polynucleotide consisting of the nucleotide sequence shown in SEQ ID NO: 7 in the sequence listing,
   (b) a polynucleotide consisting of the nucleotide sequence shown in SEQ ID NO: 8 in the sequence listing,
   (c) a polynucleotide consisting of the nucleotide sequence shown in SEQ ID NO: 9 in the sequence listing,
   (d) a polynucleotide consisting of a nucleotide sequence having 95% or higher identity to the nucleotide sequence of any one of the polynucleotides (a) to (c), the polynucleotide having foreign gene expression-enhancing activity, and
   (e) a polynucleotide consisting of a nucleotide sequence having 99% or higher identity to the nucleotide sequence of any one of the polynucleotides (a) to (c), the polynucleotide having foreign gene expression-enhancing activity.
(17) A transfected cell into which the foreign gene expression vector according to the above (15) or (16) has been introduced.
(18) A transfected cell according to the above (17), wherein the cell is a cultured cell derived from a mammal.
(19) The transfected cell according to the above (18), wherein the cultured cell derived from a mammal is a COS-1 cell, a 293 cell, or a CHO cell.
(20) A method for producing a foreign gene-derived protein, comprising culturing the transfected cell according to any one of the above (17) to (19), and obtaining the foreign gene-derived protein from the culture.
(21) Use of the polynucleotide according to any one of the above (1) to (10) for the purpose of expressing a foreign gene in a transfected cell.
(22) Use of the foreign gene expression vector according to the above (15) or (16) for the purpose of expressing a foreign gene in a transfected cell.

### Advantageous Effects of Invention

The transfection of mammalian host cells with a foreign gene expression vector having the Hspa8 gene-derived promoter of the present invention is capable of markedly enhancing the expression of a foreign gene such as one encoding a therapeutic protein or an antibody. Furthermore, the combination of the promoter of the present invention with a DNA element can further enhance the expression of a foreign gene encoding a therapeutic protein, an antibody, or the like.

### Brief Description of Drawings

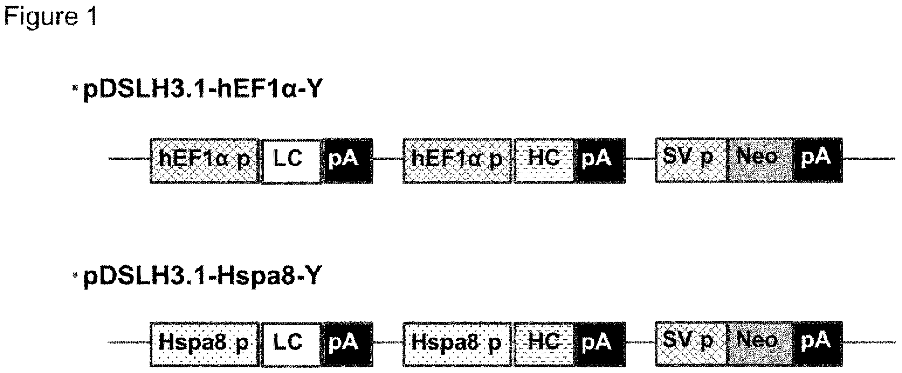

[Figure 1] Figure 1 schematically shows humanized antibody gene Y expression vectors pDSLH3.1-Hspa8-Y and pDSLH3.1-hEF1α-Y containing a Hspa8 gene or human EF1-α gene-derived promoter as a promoter for antibody H chain and L chain gene expression.

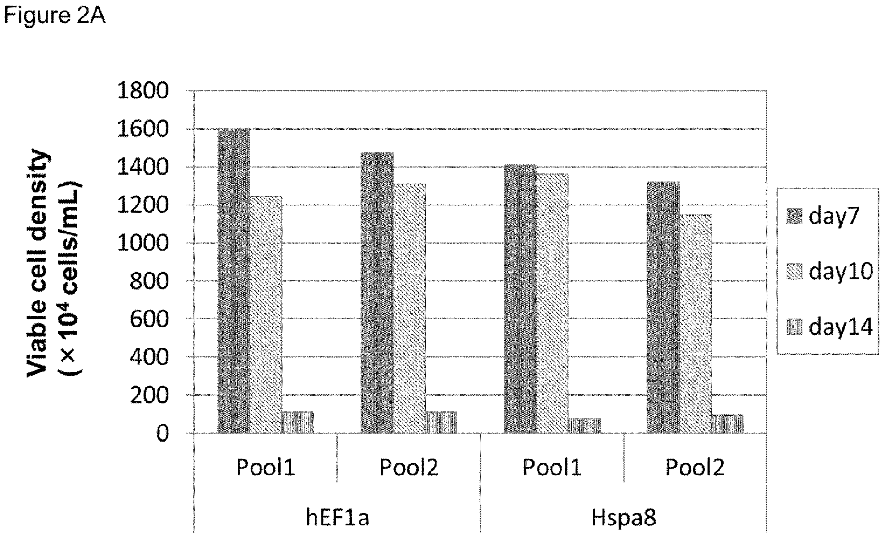

[Figure 2A] The amount of antibody produced in fed-batch culture using a humanized antibody Y-expressing stable pool was compared between expression under a Hspa8 gene promoter and expression under a human EF1-α gene promoter. Figure 2A shows the number of viable cells on each day of sampling.

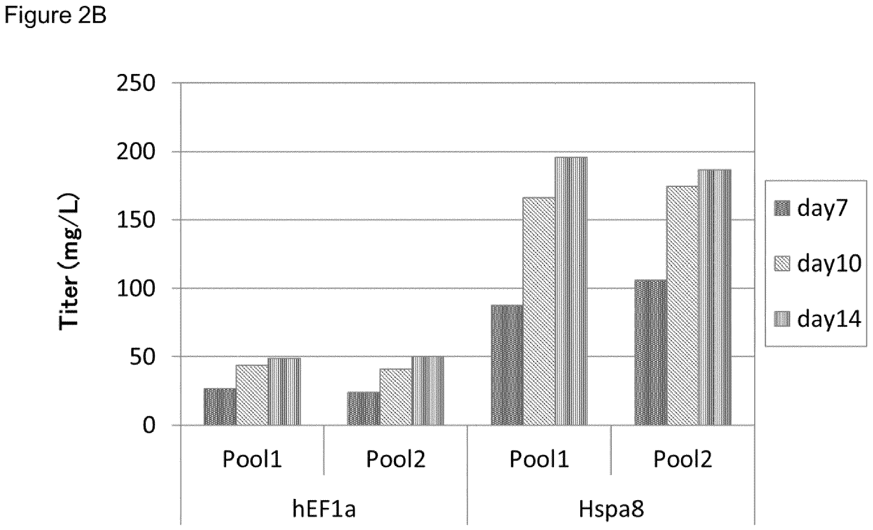

[Figure 2B] The amount of antibody produced in fed-batch culture using a humanized antibody Y-expressing stable pool was compared between expression under a Hspa8 gene promoter and expression under a human EF1-α gene promoter. Figure 2B shows the amount of the antibody produced on each day of sampling.

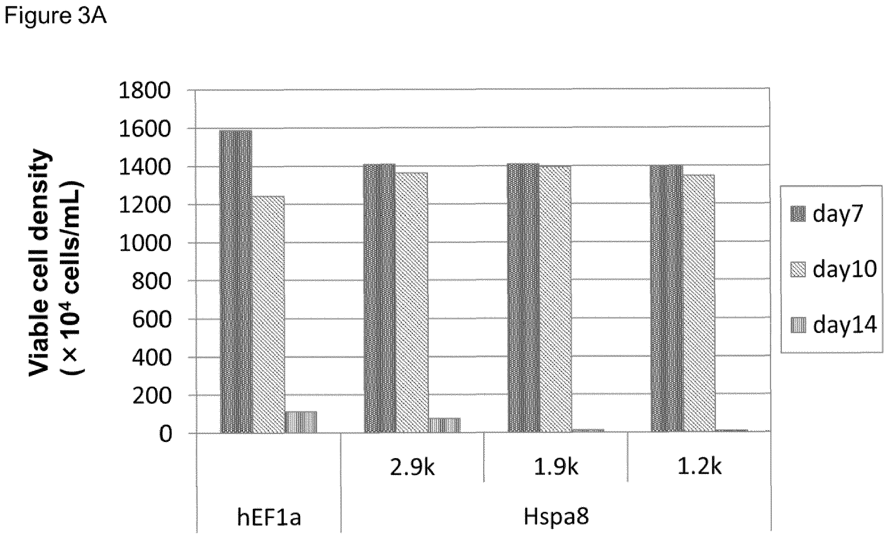

[Figure 3A] The amount of antibody produced in the fed-batch culture of a humanized antibody Y-expressing stable pool generated using a Hspa8 gene promoter of each promoter length was compared with that for a human EF1-α gene promoter. Figure 3A shows the number of viable cells on each day of sampling.

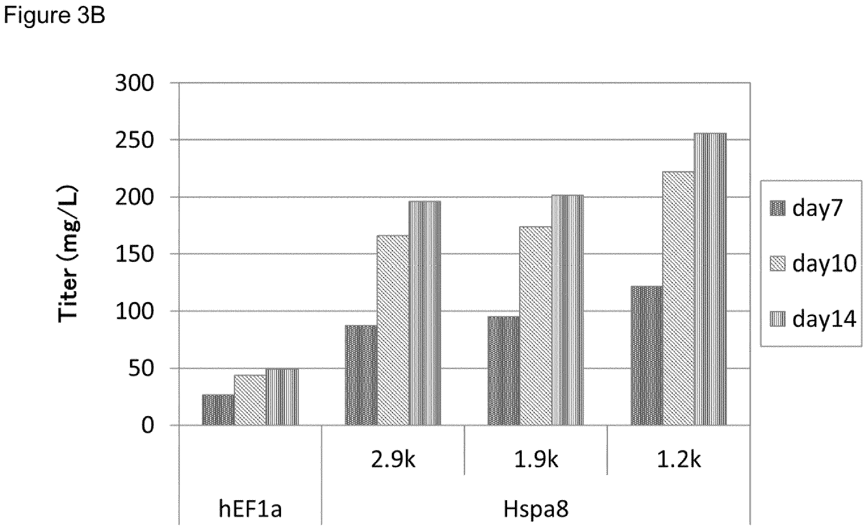

[Figure 3B] The amount of antibody produced in the fed-batch culture of a humanized antibody Y-expressing stable pool generated using a Hspa8 gene promoter of each promoter length was compared with that for a human EF1-α gene promoter. Figure 3B shows the amount of antibody produced on each day of sampling.

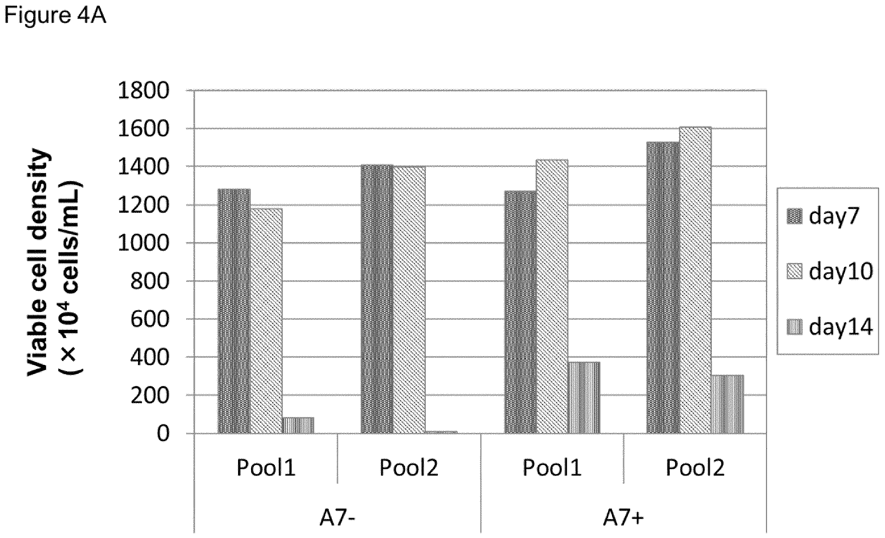

[Figure 4A] The amount of antibody produced in fed-batch culture using a stable pool generated by a humanized antibody gene Y expression vector using the Hspa8 gene as a promoter for antibody H chain and L chain gene expression and either containing or not containing the DNA element A7 was compared in order to compare between the presence and absence of the DNA element A7. Figure 4A shows the number of viable cells on each day of sampling.

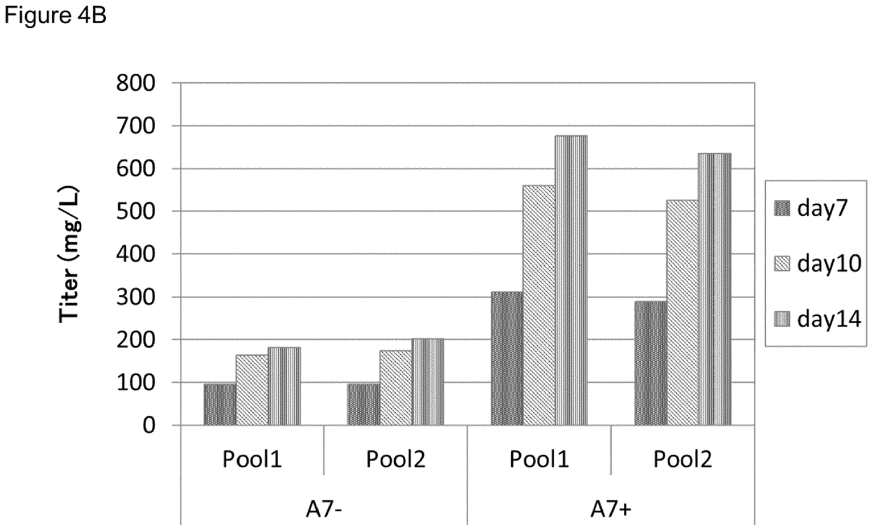

[Figure 4B] The amount of antibody produced in fed-batch culture using a stable pool generated by a humanized antibody gene Y expression vector using the Hspa8 gene as a promoter for antibody H chain and L chain gene expression and using a humanized antibody gene Y expression vector either containing or not containing the DNA element A7 was compared in order to compare between the presence and absence of the DNA element A7. Figure 4B shows the amount of antibody produced on each day of sampling.

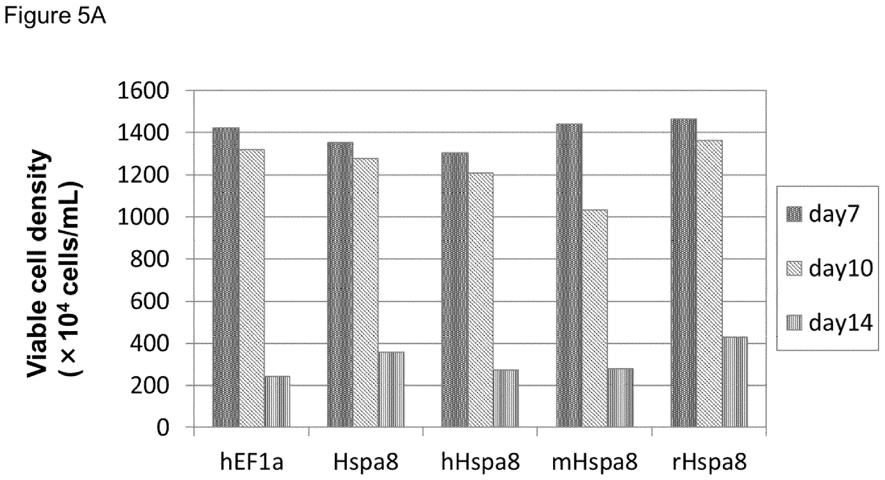

[Figure 5A] The amount of antibody produced in the fed-batch culture of a humanized antibody Y-expressing stable pool generated using a Hspa8 gene promoter was compared between species from which the Hspa8 gene promoter was derived. Hspa8, hHspa8, mHspa8, and rHspa8 represent results of the Hspa8 derived from Chinese hamster, human, mouse, and rat, respectively. Figure 5A shows the number of viable cells on each day of sampling.

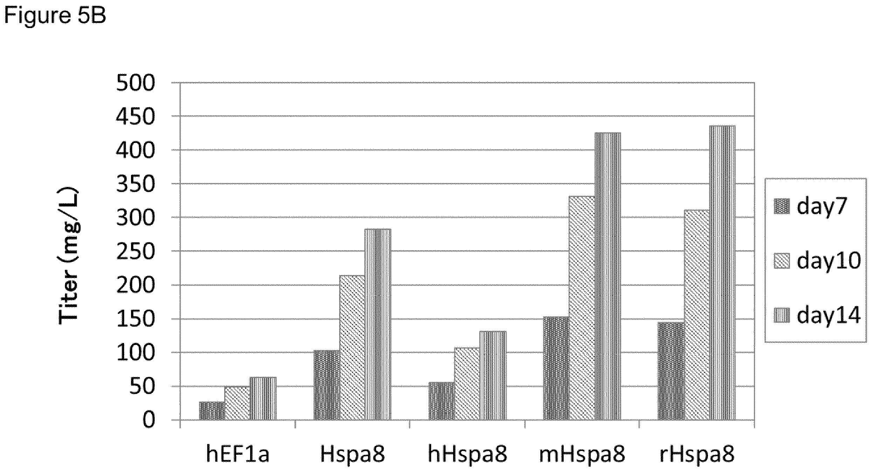

[Figure 5B] The amount of antibody produced in the fed-batch culture of a humanized antibody Y-expressing stable pool generated using a Hspa8 gene promoter was compared between species from which the Hspa8 gene promoter was derived. Hspa8, hHspa8, mHspa8, and rHspa8 represent results of the Hspa8 derived from Chinese hamster, human, mouse, and rat, respectively. Figure 5B shows the amount of antibody produced on each day of sampling.

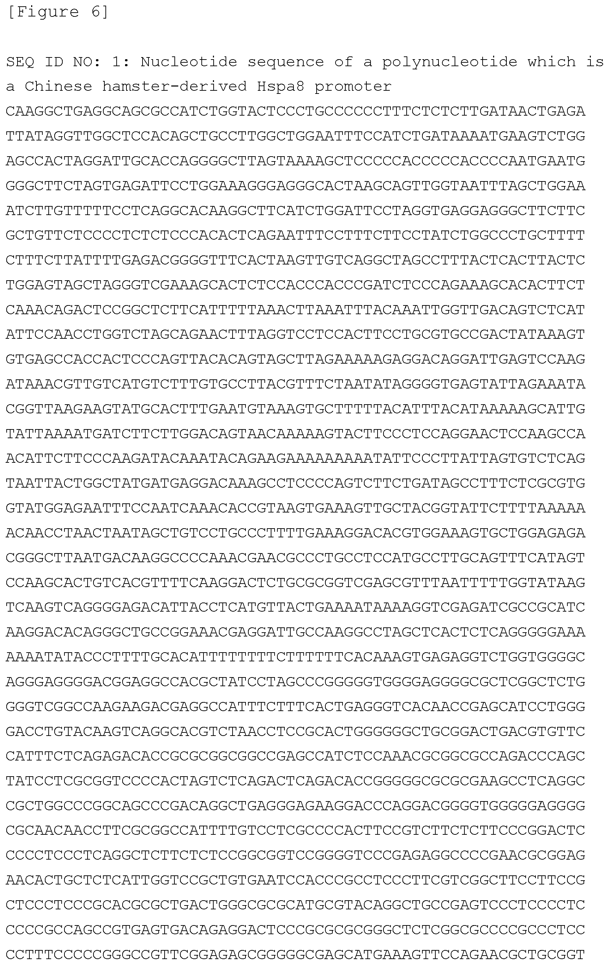

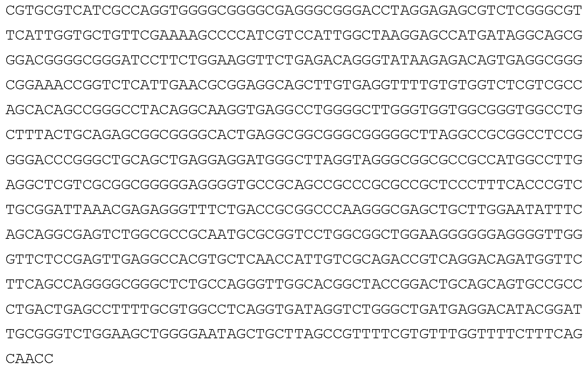

[Figure 6] Figure 6 shows the nucleotide sequence of a polynucleotide which is a Chinese hamster-derived Hspa8 gene promoter.

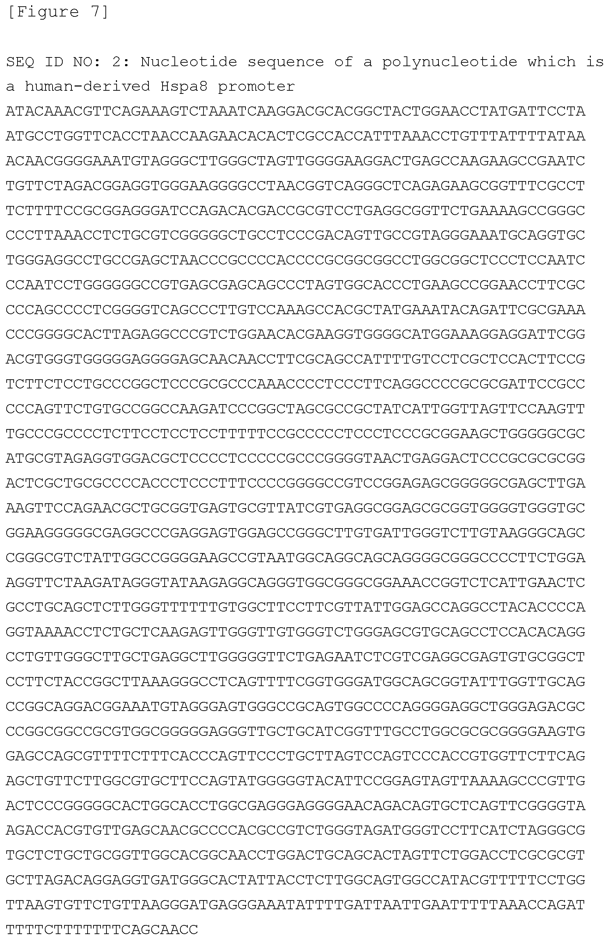

[Figure 7] Figure 7 shows the nucleotide sequence of a polynucleotide which is a human-derived Hspa8 gene promoter.

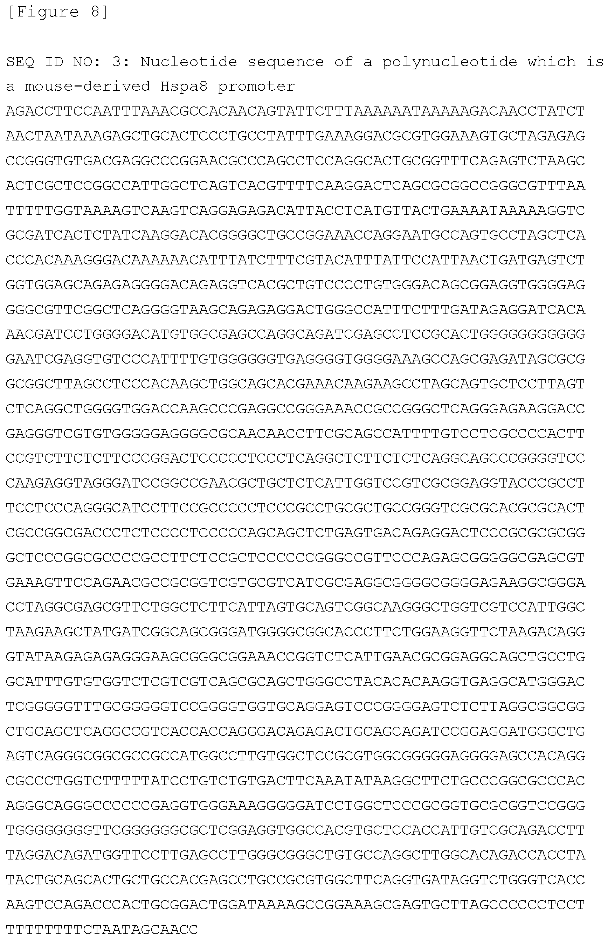

[Figure 8] Figure 8 shows the nucleotide sequence of a polynucleotide which is a mouse-derived Hspa8 gene promoter.

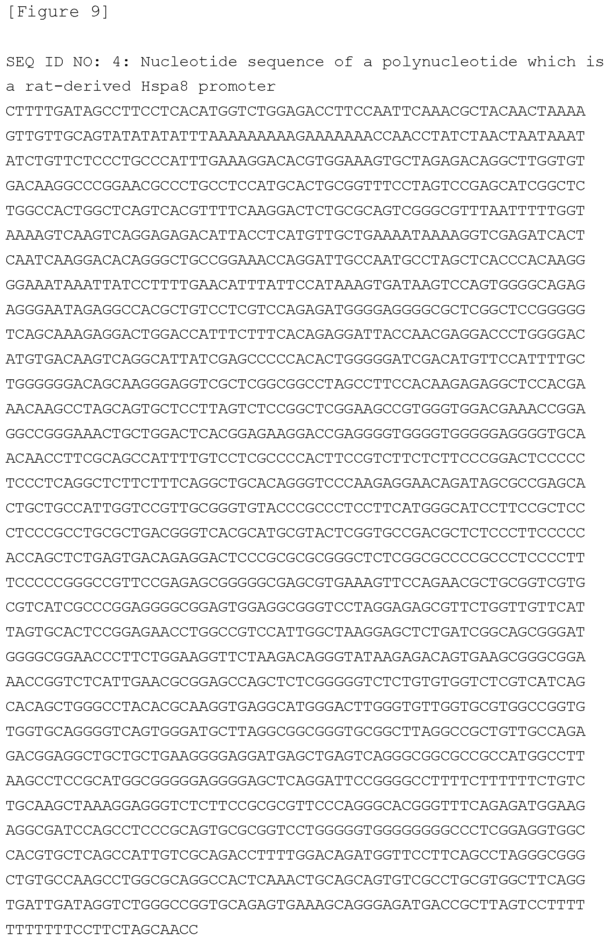

[Figure 9] Figure 9 shows the nucleotide sequence of a polynucleotide which is a rat-derived Hspa8 gene promoter.

### Description of Embodiments

Hereinafter, the present invention will be specifically described.

In the present description, the term "gene" means a moiety that is transcribed into mRNA and translated into a protein, and is used to include, not only DNA but also its mRNA and cDNA, and the RNA thereof.

In the present description, the term "polynucleotide" is used to have the same meaning as that of a nucleic acid, and includes DNA, RNA, a probe, an oligonucleotide, and a primer.

In the present description, the term "polypeptide" is used without being distinguished from the term "protein".

In the present description, the term "gene expression" means a phenomenon in which a gene is transcribed into mRNA, and/or a phenomenon in which the mRNA is translated into a protein.

In the present description, the term "foreign gene" means a gene that is artificially introduced into host cells.

In the present description, the term "foreign protein" means a protein encoded by the foreign gene.

In the present description, the term "gene expression unit" means a polynucleotide having at least a promoter region, a foreign gene, and a transcriptional terminator region (polyA addition signal) in the reading frame direction of transcription.

In the present description, the term "promoter" means a region to which a transcription factor involved in the start of transcription of DNA into RNA binds. In the present description, the term "promoter region" is also used. Examples of the promoter can include a polynucleotide from a nucleotide approximately 3 kbp upstream of a start codon to a nucleotide immediately upstream of a nucleotide sequence corresponding to the start codon. The promoter may contain 5'UTR and an intron.

In the present description, the term "promoter activity" refers to activity by which transcription starts through the binding of a transcription factor to the promoter to perform the production of a protein encoded by the gene. Promoter activity can be examined by using, as an indicator, the activity of a protein encoded by a reporter gene, such as firefly luciferase.

In the present description, the phrase "to have promoter activity" means that an antibody expression level equivalent to or higher than that of a human EF-1α gene promoter is exhibited, under conditions similar to those for the evaluation of promoter activity with antibody expression level as an indicator in fed-batch culture as described in (Example 2) mentioned later.

In the present description, the term "DNA element" means a polynucleotide having foreign gene expression-enhancing activity when located in proximity to a gene expression unit or in a foreign gene expression vector comprising the gene expression unit.

In the present description, the term "antigen-binding fragment of the antibody" means a partial fragment of the antibody having binding activity to the antigen. Examples thereof include Fab and F(ab')₂, though the antigen-binding fragment is not limited to these molecules as long as it has antigen-binding ability.

In the present description, the term "identity" refers to the relationship between sequences as to two or more nucleotide sequences or amino acid sequences and is determined by the comparison of the sequences, as known in the art. The term "identity" in the art also means, in some cases, the degree to which the sequence of a nucleic acid molecule or a polypeptide matches when comparing between two or more nucleotide sequences or between two or more amino acid sequences. The term "identity" can be evaluated by calculating percentage identity between a smaller sequence of two or more sequences and gap alignment (if present) addressed by a particular mathematical model or computer program (i.e., "algorithm"). Specifically, the identity can be evaluated using software such as ClustalW2 provided by European Molecular Biology Laboratory-European Bioinformatics Institute (EMBL-EBI), though the evaluation method is not limited thereto as long as the method is used by a person skilled in the art.

In the present description, the phrase "to hybridize under stringent conditions" refers to hybridization under conditions in which a so-called specific hybrid is formed whereas a non-specific hybrid is not formed. Examples thereof can include conditions under which a complementary strand of a nucleic acid consisting of a nucleotide sequence having 80% or higher, preferably 90% or higher, more preferably 95% or higher, most preferably 99% or higher identity to a nucleic acid hybridizes whereas a complementary strand of a nucleic acid consisting of a nucleotide sequence having lower identity does not hybridize. More specifically, the phrase is used to mean that hybridization is carried out in the commercially available hybridization solution ExpressHyb Hybridization Solution (manufactured by Clontech) at 68°C, or that hybridization is carried out under conditions using a DNA-immobilized filter in the presence of 0.7 to 1.0 M NaCl at 68°C, and the resultant is then washed at 68°C with a 0.1 × to 2 × SSC solution (wherein 1 × SSC consists of 150 mM NaCl and 15 mM sodium citrate), or conditions equivalent thereto.

### 1. Promoter for use in enhancement of foreign gene expression

The promoter for use in the enhancement of foreign gene expression according to the present invention (hereinafter, also referred to as the "promoter of the present invention") is a promoter of a heat-shock protein A8 gene (hereinafter, referred to as "Hspa8"). The promoter is not particularly limited as long as the promoter is a polynucleotide having activity as a Hspa8 promoter. The Hspa8 promoter is preferably a polynucleotide from a nucleotide approximately 2.9 kbp upstream of a start codon to a nucleotide immediately upstream of a nucleotide sequence corresponding to the start codon.

The origin of the Hspa8 promoter is not particularly limited and may be of mammalian origin. Examples thereof can include Chinese hamster, human, mouse, and rat derived Hspa8 promoters. The promoter of the present invention is preferably a rodent-derived Hspa8 promoter, more preferably a Chinese hamster-, mouse-, or rat-derived Hspa8 promoter, still more preferably a mouse- or rat-derived Hspa8 promoter. Examples of a Chinese hamster-derived Hspa8 promoter include the polynucleotide shown in SEQ ID NO: 1 in the sequence listing and Figure 6. The nucleotide sequence of SEQ ID NO: 1 is a sequence from a nucleotide approximately 2.9 kbp upstream of the start codon of the Chinese hamster-derived Hspa8 gene to the nucleotide immediately upstream of the nucleotide sequence corresponding to the start codon.

The nucleotide sequences of SEQ ID NOs: 2, 3, and 4 are sequences from a nucleotide approximately 2 kbp upstream of the start codon of human-derived Hspa8, mouse-derived Hspa8, and rat-derived Hspa8, respectively, to the nucleotide immediately upstream of the nucleotide sequence corresponding to the start codon. The nucleotide sequences of SEQ ID NOs: 2, 3, and 4 are also shown in Figures 7, 8, and 9, respectively.

The Chinese hamster-derived Hspa8 promoter may have a nucleotide sequence consisting of a partial sequence of the sequence shown in SEQ ID NO: 1. Examples thereof include polynucleotides comprising the sequences shown in SEQ ID NOs: 5 and 6, which are sequences from a nucleotide approximately 1.9 and 1.2 kbp, respectively, upstream of the start codon of Hspa8 to the nucleotide immediately upstream of the nucleotide sequence corresponding to the start codon, and further include polynucleotides comprising sequences from a nucleotide approximately 1.1 and 1.0 kbp, respectively, upstream of the start codon of Hspa8 to the nucleotide immediately upstream of the nucleotide sequence corresponding to the start codon. The polynucleotide shown in SEQ ID NO: 6 is preferred.

The promoter of the present invention may be a polynucleotide consisting of a nucleotide sequence having 80% or higher, preferably 90% or higher, more preferably 95% or higher, most preferably 99% or higher identity to the nucleotide sequence shown in any one of SEQ ID NOs: 1 to 6, and having promoter activity.

The promoter of the present invention may be a polynucleotide that hybridizes under stringent conditions to a polynucleotide consisting of a nucleotide sequence complementary to a polynucleotide consisting of any one nucleotide sequence selected from the group consisting of the nucleotide sequences shown in SEQ ID NOs: 1 to 6, and having promoter activity.

The promoter of the present invention may be a mutant polynucleotide consisting of a nucleotide sequence comprising a deletion, substitution, and/or addition of one or more, preferably 1 to 300, more preferably 1 to 30 nucleotides in any one nucleotide sequence selected from the group consisting of the nucleotide sequences shown in SEQ ID NOs: 1 to 6, and having promoter activity.

The introduction of a mutation (deletion, substitution, and/or addition) into the nucleotide sequence can be performed by an approach known in the art such as the Kunkel method or the Gapped-Duplex method, or a method equivalent thereto. For example, a kit for mutation introduction which exploits site-directed mutagenesis (e.g., Mutant-K (manufactured by Takara Bio Inc.) or Mutant-G (manufactured by Takara Bio Inc.), or an LA PCR *in vitro* Mutagenesis series kit from Takara Bio Inc.) can be utilized. Such a mutant polynucleotide can also be used as the promoter of the present invention.

The foreign gene expression-enhancing activity possessed by the promoter of the present invention can be examined by using, as an indicator, the activity of a protein encoded by a reporter gene, such as firefly luciferase, or the amount of an antibody produced in fed-batch culture. When the amount of the antibody produced in fed-batch culture is equivalent or higher, preferably 1.2 or more times, more preferably 1.5 or more times higher by use of the promoter of the present invention compared with use of a human EF-1α promoter, it can be determined that this promoter has foreign gene expression-enhancing activity. Even in the case of an enhancement of approximately 1.2-fold or more, a reduction of cell culture scale, culture time and the number of purification steps is expected. As a result, an improvement in yield and a reduction in culture cost are attained. The improved yield permits stable supply of a foreign protein as a medicament. Also, the reduced culture cost leads to a reduction in the prime cost of a foreign protein as a medicament.

### 2. Foreign gene expression unit

The foreign gene expression unit of the present invention (hereinafter, also referred to as the "gene expression unit of the present invention") has at least the promoter of the present invention described in the preceding section 1., a foreign gene, and a transcriptional terminator region (polyA addition signal) in the reading frame direction of transcription.

The polyA addition sequence can be any sequence having the activity of terminating transcription from the promoter, and may be derived from a gene which is the same as or different from the gene of the promoter.

### 3. DNA element for use in enhancing foreign gene expression

Combined use of the gene expression unit of the present invention described in the preceding section 2. with a DNA element can further enhance the expression of a foreign gene. The DNA element for combined use can be obtained by interaction with acetylated histone H3 as an indicator. In general, the acetylation of histone (H3, H4) is reportedly involved in the activation of transcription on the basis of two main hypotheses: that conformational change of the nucleosome is involved such that histone tail acetylation neutralizes the charge thereof to loosen the binding between the DNA and the histone (Mellor J. (2006) Dynamic nucleosomes and gene transcription. Trends Genet. 22 (6): 320-329); and that the acetylation is involved in the recruitment of various transcription factors (Nakatani Y. (2001) Histone acetylases - versatile players. Genes Cells. 6 (2): 79-86). Both of the hypotheses strongly suggest that the acetylation of histone is involved in transcriptional activation. Thus, chromatin immunoprecipitation (ChIP) using an anti-acetylated histone H3 antibody is capable of enriching a sample for a DNA element that interacts with acetylated histone H3.

Examples of the DNA element for use in the enhancing of foreign gene expression in combination with the promoter of the present invention can include A2, A7, and A18.

A2 is positioned at a site from 80966429 to 80974878 of human chromosome 15 and is an 8450 bp polynucleotide having an AT content of 62.2%. The nucleotide sequence of A2 is shown in SEQ ID NO: 7 in the sequence listing.

A7 is positioned at a site from 88992123 to 89000542 of human chromosome 11 and is an 8420 bp polynucleotide having an AT content of 64.52%. The nucleotide sequence of A7 is shown in SEQ ID NO: 8 in the sequence listing.

A18 is positioned at a site from 111275976 to 111284450 of human chromosome 4 and is an 8475 bp polynucleotide having an AT content of 62.54%. The nucleotide sequence of A18 is shown in SEQ ID NO: 9 in the sequence listing.

The foreign gene expression-enhancing activity possessed by the DNA element for combined use with the promoter of the present invention can be examined by using, as an indicator, the activity of a protein encoded by a reporter gene, such as SEAP.

For combined use with the promoter of the present invention, any one of the DNA elements described above may be used alone, or two or more copies of one DNA element may be used. Alternatively, two or more DNA elements may be used in combination.

The DNA element used in the present invention may consist of a nucleotide sequence having 80% or higher, preferably 90% or higher, more preferably 95% or higher, most preferably 99% or higher identity to the nucleotide sequence shown in any of SEQ ID NOs: 7 to 9, and having foreign gene expression-enhancing activity. A homology search of the nucleotide sequence can be performed using, for example, a program such as FASTA or BLAST and the DNA Data Bank of JAPAN as the subject of the search.

A person skilled in the art can readily obtain such a homolog gene of the promoter of the present invention with reference to Molecular Cloning (Sambrook, J. et al., Molecular Cloning: a Laboratory Manual 2nd ed., Cold Spring Harbor Laboratory Press, 10 Skyline Drive Plainview, NY (1989)), etc. Likewise, the identity of the nucleotide sequence described above can be determined by FASTA search or BLAST search.

The introduction of a mutation (deletion, substitution, and/or addition) into the polynucleotide can be performed by an approach known in the art such as the Kunkel method or the Gapped-Duplex method, or a method equivalent thereto. For example, a kit for mutation introduction (e.g., Mutant-K (manufactured by Takara Bio Inc.) or Mutant-G (manufactured by Takara Bio Inc.), or an LA PCR *in vitro* Mutagenesis series kit from Takara Bio Inc.) can be utilized which exploits site-directed mutagenesis. Such a mutant polynucleotide can also be used as the DNA element of the present invention.

### 4. Obtaining the polynucleotide

In the present invention, a polynucleotide comprising a foreign gene encoding a foreign protein whose production is to be enhanced as mentioned later can be obtained by a general method given below. The polynucleotide can be isolated, for example, by screening a cDNA library derived from cells or tissues expressing the foreign gene, using a DNA probe synthesized on the basis of the gene fragment. mRNA can be prepared by an approach usually used in the art. For example, the cells or the tissues are treated with a guanidine reagent, a phenol reagent, or the like to obtain total RNA. Then, poly(A) + RNA (mRNA) is obtained therefrom by the affinity column method using an oligo(dT) cellulose column, polyU-Sepharose with Sepharose 2B as a carrier, or the like, or by the batch method. The poly(A) + RNA may be further fractionated by the sucrose density gradient centrifugation method or the like. Subsequently, single-stranded cDNA is synthesized with the obtained mRNA as a template using an oligo dT primer and reverse transcriptase. Double-stranded cDNA is synthesized from the single-stranded cDNA using DNA synthetase I, DNA ligase and RNase H, etc. The synthesized double-stranded cDNA is converted into a blunt-ended form with T4 DNA synthetase, then subjected to the linkage of an adaptor (e.g., an EcoRI adaptor), phosphorylation, etc., and incorporated into a λ phage such as λgt11 for *in vivo* packaging to prepare a cDNA library. Alternatively, the cDNA library may be prepared using a plasmid vector instead of the λ phage. Then, a clone having the DNA of interest (a positive clone) can be selected from the cDNA library.

In the case of isolating a polynucleotide comprising the promoter and a terminator region, the DNA element, or a polynucleotide comprising a foreign gene for use in protein production from genomic DNA, the genomic DNA is extracted from a cell line of an organism serving as a source, followed by polynucleotide selection, according to a general approach (Molecular Cloning (1989) and Methods in Enzymology 194 (1991)). The extraction of the genomic DNA can be performed according to, for example, the method of Cryer et al. (Methods in Cell Biology, 12, 39-44 (1975)) and the method of P. Philippsen et al. (Methods Enzymol., 194, 169-182 (1991)).

The obtaining of the polynucleotide of interest comprising the promoter, the DNA element, or a foreign gene can also be performed by, for example, PCR (PCR Technology. Henry A. Erlich, Atockton press (1989)). The amplification of the polynucleotide by PCR employs a 20 to 30 mer synthetic single-stranded DNA as a primer and genomic DNA as a template. The amplified gene is used after its polynucleotide sequence is confirmed. A genomic DNA library such as a bacterial artificial chromosome (BAC) library may be used as a template for PCR.

On the other hand, a polynucleotide comprising a foreign gene having an unknown sequence can be obtained by (a) preparing a gene library according to a common method, (b) selecting the desired polynucleotide from the prepared gene library, and amplifying the polynucleotide. The gene library can be prepared by partially digesting chromosomal DNA obtained by a common method from a cell line of an organism serving as a source, with an appropriate restriction enzyme to prepare fragments, ligating the obtained fragments into an appropriate vector, and introducing the vector into an appropriate host. Alternatively, the gene library may be prepared by extracting mRNA from the cells, synthesizing cDNA therefrom, then ligating the cDNA into an appropriate vector, and introducing the vector into an appropriate host. In this respect, a plasmid known as a well-known vector for gene library preparation can be used as the vector, and a phage vector or a cosmid, etc. can also be widely used. The host to be transfected or transduced can be used according to the type of vector. The polynucleotide comprising a foreign gene is selected from the gene library by colony hybridization, plaque hybridization, or the like using a labeled probe comprising a sequence unique to the foreign gene.

The polynucleotide comprising a foreign gene can also be synthesized entirely chemically. The gene can be synthesized by, for example, a method of preparing a pair of complementary oligonucleotides and annealing these, a method of ligating several annealed DNAs using DNA ligase, or a method of preparing several partially complementary oligonucleotides and filling the gaps therein by PCR.

The polynucleotide sequence can be determined by a usual method, for example, the dideoxy method (Sanger et al., Proc. Natl. Acad. Sci., USA, 74, 5463-5467 (1977)). Alternatively, the polynucleotide sequence may be readily determined using a commercially available sequencing kit or the like.

### 5. Foreign gene expression vector

A vector comprising the foreign gene expression unit described in the preceding section 2. comprising the promoter described in the preceding section 1. is provided as a foreign gene expression vector of the present invention. The foreign gene expression vector of the present invention may comprise one of the DNA elements described in the preceding section 3., two or more copies of one DNA element, or a combination of two or more DNA elements. When a foreign gene is expressed in host cells using the foreign gene expression vector, the DNA element may be located immediately preceding or immediately following the gene expression unit, or may be located at a position distant from the gene expression unit. Alternatively, one foreign gene expression vector comprising a plurality of DNA elements may be used. The orientation of the DNA element may be in either the forward direction or the reverse direction with respect to the gene expression unit.

Examples of the foreign gene can include, but are not particularly limited to: reporter genes such as the secreted alkaline phosphatase (SEAP) gene, the green fluorescence protein (GFP) gene, and the luciferase gene; various enzyme genes such as the α-amylase gene and the α-galactosidase gene; genes of various interferons such as interferon α and interferon γ, which are pharmaceutically useful physiologically active proteins; genes of various interleukins such as IL1 and IL2; various cytokine genes such as the erythropoietin (EPO) gene and the granulocyte colony-stimulating factor (G-CSF) gene; growth factor genes; and a gene encoding a multimeric protein, for example, a gene encoding a heteromultimer which is an antibody or an antigen-binding fragment thereof. These genes may be obtained by any approach.

The term "antigen-binding fragment of the antibody" means a partial fragment of the antibody having binding activity to the antigen. Examples thereof include Fab, F(ab')₂, Fv, scFv, a diabody, linear antibodies, and multispecific antibodies formed from antibody fragments. Also, Fab', which is a monovalent fragment of antibody variable regions obtained by the treatment of F(ab')₂ under reductive conditions is included within the antigen-binding fragment of the antibody. However, the antigen-binding fragment is not limited to these molecules as long as it has antigen-binding ability. Furthermore, these antigen-binding fragments also include, not only a fragment obtained by the treatment of the full-length molecule of an antibody protein with an appropriate enzyme but also a protein produced in appropriate host cells using a genetically engineered antibody gene.

The foreign gene expression vector of the present invention can comprise a selection marker for selecting a transfectant. The transfectant can be selected using, for example, a drug resistance marker which confers resistance to a drug such as cerulenin, aureobasidin, zeocin, canavanine, cycloheximide, hygromycin, puromycin, blasticidin, tetracycline, kanamycin, ampicillin, or neomycin. Alternatively, the transfectant may be selected by using, as a marker, a gene that confers, for example, solvent resistance to ethanol or the like, osmotic pressure resistance to glycerol, a salt, or the like, or metal ion resistance to copper or the like.

The foreign gene expression vector of the present invention may be a vector that is not integrated into chromosomal DNA. In general, the foreign gene expression vector is randomly integrated into the chromosome after transfection of host cells. By contrast, use of a constituent derived from a mammalian virus such as simian virus 40 (SV40), papillomavirus (BPV, HPV), or EBV allows the foreign gene expression vector to be used as an episomal vector capable of replicating autonomously in the transfected host cells. For example, a vector having a sequence encoding a SV40-derived replication origin and a trans-acting factor SV40 large T antigen, or a vector having a sequence encoding EBV-derived oriP and EBNA-1 is widely used. The DNA element is capable of exhibiting foreign gene expression-enhancing activity, regardless of the type of vector or the presence or absence of integration into the chromosome.

### 6. Transfected cells

The transfected cells of the present invention are transfected cells comprising the foreign gene expression vector of the preceding section 5. introduced thereinto.

The host cells to be transfected are eukaryotic cells, preferably mammalian cells, more preferably human-, mouse-, rat-, hamster-, monkey-, or bovine-derived cells. Examples of the mammalian cells can include, but are not limited to, COS-1 cells, 293 cells, and CHO cells (CHO-K1, CHO-O1, CHO DG44, CHO dhfr-, CHO-S) .

In the present invention, the method for introducing the expression vector into host cells can be any method as long as the method allows the introduced gene to be present stably in the host and to be appropriately expressed. Examples thereof can include methods generally used, for example, the calcium phosphate method (Ito et al., (1984) Agric. Biol. Chem., 48, 341), electroporation (Becker, D.M. et al. (1990) Methods. Enzymol., 194, 182-187), the spheroplast method (Creggh et al., Mol. Cell. Biol., 5, 3376 (1985)), the lithium acetate method (Itoh, H. (1983) J. Bacteriol. 153, 163-168), and lipofection.

### 7. Method for producing foreign protein

The method for producing a foreign protein according to the present invention can be performed by culturing the transfected cells described in the preceding section 6. by a known method, and collecting the foreign protein from the culture, followed by purification. The "culture" means any of a culture supernatant, cultured cells, and a cell homogenate. Not only a monomeric protein but also a multimeric protein may be selected as the foreign protein that can be produced using the transfected cells described in section 6. In the case of producing a heteromultimeric protein constituted by a plurality of different subunits, a plurality of genes encoding these subunits each need to be introduced into the host cells described in section 6.

The method for culturing the transfected cells can be performed according to a usual method for use in the culture of the host cells.

When the transfected cells are mammalian cells, the transfected cells are cultured, for example, at 37°C under 5% or 8% CO₂ conditions for a culture time on the order of 24 to 1000 hours. The culture can be carried out by, for example, static culture, shake culture, stirring culture, batch culture under aeration, fed-batch culture, perfusion culture or continuous culture.

The expression product of the foreign protein gene from the culture (culture solution) described above can be confirmed by SDS-PAGE, Western blotting, ELISA, or the like.

### 8. Method for producing antibody protein

Examples of the heteromultimeric protein to be produced using the production method described in the preceding section 7. can include antibody proteins. The antibody protein is a tetramer protein consisting of two molecules of a heavy chain polypeptide and two molecules of a light chain polypeptide. Thus, for obtaining an antibody protein in a form that maintains antigen-binding ability, it is necessary to introduce both heavy chain and light chain genes into the transfected cells described in the preceding section 6. In this case, heavy chain and light chain gene expression units may be present on the same expression vector or may be present on different expression vectors.

Examples of the antibody to be produced according to the present invention can include antibodies prepared by immunizing laboratory animals such as rabbits, mice, and rats with the desired antigen. Further examples of the antibody to be produced according to the present invention can include chimeric antibodies and humanized antibodies originating from the antibodies described above. In addition, a human antibody obtained from a genetically engineered animal or by the phage display method is also an antibody to be produced according to the present invention.

The antibody gene for use in antibody production is not limited to an antibody gene having a particular polynucleotide sequence as long as a combination of a heavy chain polypeptide and a light chain polypeptide obtained by the transcription of the antibody gene and subsequent translation retains the activity of binding to a given antigen protein.

The antibody gene is not necessarily required to encode the full-length molecule of the antibody. A gene encoding an antigen-binding fragment of the antibody can be used. The gene encoding such an antigen-binding fragment can be obtained by genetically engineering a gene encoding the full-length molecule of the antibody protein.

### 9. Methods for producing other foreign proteins

Examples of the foreign protein to be produced by the production method of the present invention can include the antibodies mentioned above as well as various human- or non-human animal-derived proteins, antigen-binding fragments thereof, and modified forms of the proteins or the fragments. Examples of such a protein and the like can include, but are not limited to: peptide hormones such as atrial natriuretic peptide (ANP), brain natriuretic peptide (BNP), C-type natriuretic peptide (CNP), vasopressin, somatostatin, growth hormone (GH), insulin, oxytocin, ghrelin, leptin, adiponectin, renin, calcitonin, osteoprotegerin, and insulin-like growth factor (IGF); cytokines such as interleukin, chemokine, interferon, tumor necrosis factor (TNFα/β as well as TNF superfamily, etc.), nerve growth factor (NGF), cell growth factor (EGF, FGF, PDGF, HGF, TGF, etc.), hematopoietic factor (CSF, G-CSF, erythropoietin, etc.), and adiponectin; receptors such as TNF receptor; enzymes such as lysozyme, protease, proteinase, and peptidase; functional fragments thereof (fragments partially or wholly retaining the biological activity of the original protein); and fusion proteins comprising these proteins.

### Examples

Hereinafter, the present invention will be specifically described with reference to Examples. However, these Examples do not limit the technical scope of the present invention by any means. Plasmids, restriction enzymes, DNA-modifying enzymes, etc. used in the Examples of the present invention are commercially available and can be used according to common methods. Operations used in DNA cloning, polynucleotide sequencing, transfection of host cells, culture of transfected cells, collection of a protein from the resulting culture, purification, etc. are also well known to a person skilled in the art or can be derived from the literature.

### (Example 1) Cloning of Hspa8 gene promoter region

The Hspa8 promoter region used was a sequence from a nucleotide approximately 2.9 kbp upstream of the start codon of Hspa8 to the nucleotide immediately upstream of the nucleotide sequence corresponding to the start codon with reference to the sequence of mRNA registered under NM_001246729.1 and the scaffold sequence of the Chinese hamster genome registered under NW_003616190.1 in GenBank.

The Hspa8 promoter region was amplified by PCR with the genomic DNA of CHO cells as a template using the primer set given below and PrimeSTAR Max DNA Polymerase (Takara Bio Inc.), and the PCR product was purified using a QIAquick PCR Purification kit (Qiagen). The nucleotide sequence of the cloned Chinese hamster Hspa8 promoter region is shown in SEQ ID NO: 1 in the sequence listing. Primer set for Hspa8 promoter:
Hspa8-NotI-F: TTCGCGGCCGCCAAGGCTGAGGCAGCG (SEQ ID No. 10)
Hspa8-XbaI-R: TTCTCTAGAGGTTGCTGAAAGAAAACCAAA (SEQ ID No. 11)

### (Example 2) Evaluation of Hspa8 promoter by fed-batch culture with antibody expression level as indicator

### 2-1) Construction of antibody expression vector

A humanized antibody gene Y expression vector pDSLH3.1-Hspa8-Y having pDSLH4.1 (see Okumura T et al., J Biosci Bioeng., 120 (3): 340-346, 2015) as a vector backbone was constructed. This vector contained the Hspa8 promoter for the expression of antibody H chain and L chain genes, and no DNA element. First, the DNA fragment amplified and purified in Example 1 was digested with NotI-XbaI and then inserted into the NotI-NheI sites of H chain gene expression vector pDSH1.1-hEFla-Y and L chain gene expression vector pDSL2.1-hEF1α-Y described in Patent Literature 4 to construct pDSH1.1-Hspa8-Y and pDSL2.1-Hspa8-Y, respectively. Next, a DNA fragment obtained by the digestion of pDSL2.1-Hspa8-Y with AatII-MluI was inserted into the AatII-MluI site of pDSH1.1-Hspa8-Y to construct pDSLH3.1-Hspa8-Y. The vectors are schematically shown in Figure 1.

### 2-2) Generation of humanized antibody Y-expressing stable pool

CHO-K1 cells (ATCC) were adapted to suspension culture in a serum-free suspension culture condition, to obtain CHO-O1 cells as host cells. The CHO-O1 cells were transfected with the antibody expression vector pDSLH3.1-Hspa8-Y constructed in (2-1) and pDSLH3.1-hEF1α-Y described in Patent Literature 4 using a transfection apparatus Neon Transfection System (Invitrogen), and cultured in 5% CO₂ at 37°C in a T25 flask. One day after the transfection, Geneticin (Life Technologies Corporation) was added thereto at a final concentration of 800 µg/mL, followed by drug selection culture for 1 week. Then, the cells were cultured in 5% CO₂ at 37°C in a 125 mL Erlenmeyer flask to obtain a humanized antibody Y-expressing stable pool.

### 2-3) Evaluation of amount of antibody produced by fed-batch culture of humanized antibody Y-expressing stable pool

Fed-batch culture was performed in a 125 mL Erlenmeyer flask using each humanized antibody Y-expressing stable pool generated in (2-2). The basal medium used was G13 (custom medium manufactured by IS Japan Co., Ltd.), and the feed medium used was F13 (custom medium manufactured by IS Japan Co., Ltd.).

The change in the number of viable cells and the change in the amount of antibody produced are shown in Figures 2A and 2B, respectively. The amount of antibody produced with the Hspa8 promoter on day 14 of culture reached 3.9 times the value of that with the human EF1-α promoter, and thus greatly exceeded the amount of antibody produced with a promoter that is currently frequently used.

### (Example 3) Study on Hspa8 promoter length with antibody expression level as an indicator in fed-batch culture

### 3-1) Construction of antibody expression vector

pDSLH3.1-Hspa8-1.9-Y and pDSLH3.1-Hspa8-1.2-Y were constructed by substituting the promoter for antibody H chain and L chain genes in the humanized antibody gene Y expression vector pDSLH3.1-hEF1α-Y with a partial sequence of the Hspa8 promoter. In these expression vectors, the partial sequence of the Hspa8 promoter used was a sequence from a nucleotide approximately 1.9 and 1.2 kbp, respectively, upstream of the start codon of Hspa8 to the nucleotide immediately upstream of the nucleotide sequence corresponding to the start codon.

pDSLH3.1-Hspa8-1.9-Y was constructed by the following method: first, the partial sequence of the Chinese hamster Hspa8 promoter was amplified by PCR with pDSH1.1-Hspa8-Y as a template using the primer set given below and PrimeSTAR Max DNA Polymerase, and the PCR product was purified using a QIAquick PCR Purification kit. The purified DNA fragment was digested with NotI-XbaI and then inserted into the NotI-NheI sites of H chain gene expression vector pDSH1.1-hEFla-Y and L chain gene expression vector pDSL2.1-hEF1α-Y to construct pDSH1.1-Hspa8-1.9-Y and pDSL2.1-Hspa8-1.9-Y, respectively. Next, a DNA fragment obtained by the digestion of pDSL2.1-Hspa8-1.9-Y with AatII-MluI was inserted into the AatII-MluI site of pDSH1.1-Hspa8-1.9-Y to construct pDSLH3.1-Hspa8-1.9-Y. pDSLH3.1-Hspa8-1.2-Y was constructed in the same way as above.
Primer set for Hspa8 promoter 1.9 kbp:
   Hspa8-NotI-1900F: TTCGCGGCCGCAACAACCTAACTAATAGCTGTCC (SEQ ID No. 12)
Hspa8-XbaI-R: TTCTCTAGAGGTTGCTGAAAGAAAACCAAA (SEQ ID No. 11)
Primer set for Hspa8 promoter 1.2 kbp:
   Hspa8-NotI-1200F: TTCGCGGCCGCAACCTTCGCGGCCATTTTGTCCTC (SEQ ID No. 13)
Hspa8-XbaI-R: TTCTCTAGAGGTTGCTGAAAGAAAACCAAA (SEQ ID No. 11)

### 3-2) Generation of humanized antibody Y-expressing stable pool

The CHO-O1 cells were transfected with the antibody expression vector pDSLH3.1-hEF1α-Y, pDSLH3.1-Hspa8-Y, pDSLH3.1-Hspa8-1.9-Y, or pDSLH3.1-Hspa8-1.2-Y constructed in (2-1) or (3-1), according to the method described in (2-2). Drug selection culture was performed to generate a humanized antibody Y-expressing stable pool.

### 3-3) Evaluation of amount of antibody produced by fed-batch culture of humanized antibody Y-expressing stable pool

Fed-batch culture was performed in a 125 mL Erlenmeyer flask using each humanized antibody Y-expressing stable pool generated in (3-2). The basal medium used was G13, and the feed medium used was F13.

The change in the number of viable cells and the change in the amount of antibody produced are shown in Figures 3A and 3B, respectively. The Hspa8 promoter having a decreased length increased the amount of antibody produced. The value exhibited by the 1.2 kbp Hspa8 promoter was 1.3 times the value of the 2.9 kbp one and reached 5.2 times the value of the human EF1-α promoter that was used as a control. The Hspa8 promoter having the optimized length was able to exert its strong promoter activity and consequently surpassed the human EF1-α promoter in terms of the amount of antibody produced.

### (Example 4) Study on effect brought about by combination of Hspa8 promoter and A7 with antibody expression level as an indicator in fed-batch culture

### 4-1) Construction of antibody expression vector

DNA element A7 described in Patent Literature 3 was inserted upstream of the expression cassette of the antibody expression vector pDSLH3.1-Hspa8-1.9-Y constructed in (3-1) to construct pDSLHA4.1-Hspa8-1.9-Y.

### 4-2) Generation of humanized antibody Y-expressing stable pool

The CHO-O1 cells were transfected with the DNA element A7-free antibody expression vector pDSLH3.1-Hspa8-1.9-Y constructed in (3-1), or the DNA element A7-containing antibody expression vector pDSLHA4.1-Hspa8-1.9-Y constructed in (4-1), according to the method described in (2-2). Drug selection culture was performed to generate a humanized antibody Y-expressing stable pool.

### 4-3) Evaluation of amount of antibody produced by fed-batch culture of humanized antibody Y-expressing stable pool

Fed-batch culture was performed in a 125 mL Erlenmeyer flask using each humanized antibody Y-expressing stable pool generated in (4-2). The basal medium used was G13, and the feed medium used was F13.

The change in the number of viable cells and the change in the amount of antibody produced are shown in Figures 4A and 4B, respectively. On day 14 of culture, the amount of antibody produced from the A7-containing antibody expression vector was 3.4 times the value of that from the A7-free antibody expression vector. These results demonstrated that the combined use of the Hspa8 promoter with DNA element A7 can effectively achieve high production by synergistic effects.

### (Example 5) Evaluation of human, mouse, and rat Hspa8 promoters by fed-batch culture with antibody expression level as an indicator

### 5-1) Construction of antibody expression vector

pDSLH3.1-hHspa8-Y, pDSLH3.1-mHspa8-Y, and pDSLH3.1-rHspa8-Y were constructed by substituting the promoter for antibody H chain and L chain genes in the humanized antibody gene Y expression vector pDSLH3.1-hEF1α-Y with human, mouse, and rat Hspa8 promoters, respectively. In each expression vector, the Hspa8 promoter used was a sequence from a nucleotide approximately 2.0 kbp upstream of the start codon of Hspa8 to the nucleotide immediately upstream of the nucleotide sequence corresponding to the start codon. The nucleotide sequences of the cloned human, mouse, and rat Hspa8 promoters are shown in SEQ ID NOs: 2, 3, and 4, respectively, in the sequence listing.

pDSLH3.1-hHspa8-Y was constructed by the following method: first, a human Hspa8 promoter was amplified by PCR with human genomic DNA as a template using the primer set given below and PrimeSTAR Max DNA Polymerase, and the PCR product was purified using a QIAquick PCR Purification kit. The purified DNA fragment was digested with HindIII-EcoT14I or AatII-EcoT14I and then inserted into the HindIII-NheI site of the H chain gene expression vector pDSH1.1-hEF1α-Y and the AatII-NheI site of the L chain gene expression vector pDSL2.1-hEF1α-Y to construct pDSH1.1-hHspa8-Y and pDSL2.1-hHspa8-Y, respectively. Next, a DNA fragment obtained by the digestion of pDSL2.1-hHspa8-Y with AatII-HindIII was inserted into the AatII-HindIII site of pDSH1.1-hHspa8-Y to construct pDSLH3.1-hHspa8-Y. pDSLH3.1-mHspa8-Y and pDSLH3.1-rHspa8-Y were constructed in the same way as above. Primer set for human Hspa8 promoter for H chain gene expression vector insertion:
Hspa8-human-HindIII-F: GGTGAAGCTTATACAAACGTTCAGAAAGTCTAA (SEQ ID No. 14)
Hspa8-human-EcoT14I-R: GGTGCCATGGGGTTGCTGAAAAAAAGAAAAATC (SEQ ID No. 15)
Primer set for human Hspa8 promoter for L chain gene expression vector insertion:
Hspa8-human-AatII-F: GGGTGACGTCATACAAACGTTCAGAAAGTCTAA (SEQ ID No. 16)
Hspa8-human-EcoT14I-R: GGTGCCATGGGGTTGCTGAAAAAAAGAAAAATC (SEQ ID No. 15)
Primer set for mouse Hspa8 promoter:
Hspa8-mouse-NotI-F: GGGTGCGGCCGCAGACCTTCCAATTTAAACGCCAC (SEQ ID No. 17)
Hspa8-mouse-XbaI-R: GAGGTCTAGAGGTTGCTATTAGAAAAAAAAAGG (SEQ ID No. 18)
Primer set for rat Hspa8 promoter:
Hspa8-rat-NotI-F: GGTGGCGGCCGCCTTTTGATAGCCTTCCTCACATG (SEQ ID No. 19)
Hspa8-rat-NheI-R: GGTCGCTAGCGGTTGCTAGAAGGAAAAAAAAAA (SEQ ID No. 20)

### 5-2) Generation of humanized antibody Y-expressing stable pool

The CHO-O1 cells were transfected with the antibody expression vector pDSLH3.1-hEF1α-Y, pDSLH3.1-Hspa8-1.9-Y, pDSLH3.1-hHspa8-Y, pDSLH3.1-mHspa8-Y, or pDSLH3.1-rHspa8-Y constructed in (2-1), (3-1) or (5-1), according to the method described in (2-2). Drug selection culture was performed to generate a humanized antibody Y-expressing stable pool.

### 5-3) Evaluation of amount of antibody produced by fed-batch culture of humanized antibody Y-expressing stable pool

Fed-batch culture was performed in a 125 mL Erlenmeyer flask using each humanized antibody Y-expressing stable pool generated in (5-2). The basal medium used was G13, and the feed medium used was F13.

The change in the number of viable cells and the change in the amount of antibody produced are shown in Figures 5A and 5B respectively. The amounts of antibody produced with the rat, mouse, Chinese hamster, and human Hspa8 promoters on day 14 of culture reached 7.0, 6.8, 4.5, and 2.1 times the value of that with the human EF1-α promoter, respectively, and thus greatly exceeded the amount of the antibody produced with the human EF1-α promoter, regardless of organism species. The amounts of antibody produced with the rat and mouse Hspa8 promoters were further increased compared to the value for the Chinese hamster Hspa8 promoter. These results demonstrated that the Hspa8 promoter of an appropriately selected organism species can exert its strong promoter activity.

### Industrial Applicability

The transfection of mammalian host cells with a foreign gene expression unit containing the promoter of the present invention or the foreign gene expression vector of the present invention is capable of increasing the productivity of a foreign gene such as a therapeutic protein or an antibody.

### Sequence Listing Free Text

SEQ ID NO: 1 - Chinese hamster-derived Hspa8 promoter
SEQ ID NO: 2 - Human-derived Hspa8 promoter
SEQ ID NO: 3 - Mouse-derived Hspa8 promoter
SEQ ID NO: 4 - Rat-derived Hspa8 promoter
SEQ ID NO: 5 - Nucleotide sequence of Chinese hamster-derived Hspa8 promoter from a nucleotide approximately 1.9 kbp upstream of the start codon of Chinese hamster Hspa8 to the nucleotide immediately upstream of the nucleotide sequence corresponding to the start codon
SEQ ID NO: 6 - Nucleotide sequence of Chinese hamster-derived Hspa8 promoter from a nucleotide approximately 1.2 kbp upstream of the start codon of Chinese hamster Hspa8 to the nucleotide immediately upstream of the nucleotide sequence corresponding to the start codon
SEQ ID NO: 7 - Nucleotide sequence of DNA element A2
SEQ ID NO: 8 - Nucleotide sequence of DNA element A7
SEQ ID NO: 9 - Nucleotide sequence of DNA element A18
SEQ ID NO: 10 - Primer Hspa8-NotI-F for Hspa8 promoter
SEQ ID NO: 11 - Primer Hspa8-XbaI-R for Hspa8 promoter
SEQ ID NO: 12 - Primer Hspa8-NotI-1900F for Hspa8 promoter 1.9 kbp
SEQ ID NO: 13 - Primer Hspa8-NotI-1200F for Hspa8 promoter 1.2 kbp
SEQ ID NO: 14 - Primer Hspa8-human-HindIII-F for human Hspa8 promoter
SEQ ID NO: 15 - Primer Hspa8-human-EcoT14I-R for human Hspa8 promoter
SEQ ID NO: 16 - Primer Hspa8-human-AatII-F for human Hspa8 promoter
SEQ ID NO: 17 - Primer Hspa8-mouse-NotI-F for mouse Hspa8 promoter
SEQ ID NO: 18 - Primer Hspa8-mouse-XbaI-R for mouse Hspa8 promoter
SEQ ID NO: 19 - Primer Hspa8-rat-NotI-F for rat Hspa8 promoter
SEQ ID NO: 20 - Primer Hspa8-rat-NheI-R for rat Hspa8 promoter

## Claims

1. A polynucleotide consisting of the nucleotide sequence shown in SEQ ID NO: 1 or a partial sequence of the nucleotide sequence, the polynucleotide being a Chinese hamster-derived Hspa8 gene promoter and comprising a polynucleotide consisting of the nucleotide sequence shown in SEQ ID NO: 6.

2. The polynucleotide according to claim 1, which consists of the nucleotide sequence shown in SEQ ID NO: 1.

3. The polynucleotide according to claim 1, which consists of the nucleotide sequence shown in SEQ ID NO: 5.

4. The polynucleotide according to claim 1, which consists of the nucleotide sequence shown in SEQ ID NO: 6.

5. A polynucleotide consisting of the nucleotide sequence shown in SEQ ID NO: 2 in the sequence listing, the polynucleotide being a human-derived Hspa8 gene promoter.

6. A polynucleotide consisting of the nucleotide sequence shown in SEQ ID NO: 3 in the sequence listing, the polynucleotide being a mouse-derived Hspa8 gene promoter.

7. A polynucleotide consisting of the nucleotide sequence shown in SEQ ID NO: 4 in the sequence listing, the polynucleotide being a rat-derived Hspa8 gene promoter.

8. A polynucleotide consisting of a nucleotide sequence having 95% or higher identity to the nucleotide sequence according to any one of claims 1 to 7, the polynucleotide having promoter activity.

9. A polynucleotide consisting of a nucleotide sequence having 99% or higher identity to the nucleotide sequence according to any one of claims 1 to 7, the polynucleotide having promoter activity.

10. A polynucleotide that hybridizes under stringent conditions to a polynucleotide consisting of a nucleotide sequence complementary to the nucleotide sequence according to any one of claims 1 to 9, the polynucleotide having promoter activity.

11. A foreign gene expression unit comprising the polynucleotide according to any one of claims 1 to 10.

12. The foreign gene expression unit according to claim 11, wherein the foreign gene is a gene encoding a multimeric protein.

13. The foreign gene expression unit according to claim 11, wherein the foreign gene is a gene encoding a heteromultimeric protein.

14. The foreign gene expression unit according to claim 11, wherein the foreign gene is a gene encoding an antibody or an antigen-binding fragment thereof.

15. A foreign gene expression vector comprising the foreign gene expression unit according to any one of claims 11 to 14.

16. A foreign gene expression vector comprising the foreign gene expression unit according to any one of claims 11 to 14 and any one or more polynucleotides selected from polynucleotides (a) to (e) of the following group A:
group A
(a) a polynucleotide consisting of the nucleotide sequence shown in SEQ ID NO: 7 in the sequence listing,
(b) a polynucleotide consisting of the nucleotide sequence shown in SEQ ID NO: 8 in the sequence listing,
(c) a polynucleotide consisting of the nucleotide sequence shown in SEQ ID NO: 9 in the sequence listing,
(d) a polynucleotide consisting of a nucleotide sequence having 95% or higher identity to the nucleotide sequence of any one of the polynucleotides (a) to (c), the polynucleotide having foreign gene expression-enhancing activity, and
(e) a polynucleotide consisting of a nucleotide sequence having 99% or higher identity to the nucleotide sequence of any one of the polynucleotides (a) to (c), the polynucleotide having foreign gene expression-enhancing activity.

17. A transfected cell into which the foreign gene expression vector according to claim 15 or 16 has been introduced.

18. The transfected cell according to claim 17, wherein the cell is a cultured cell derived from a mammal.

19. The transfected cell according to claim 18, wherein the cultured cell derived from a mammal is a COS-1 cell, a 293 cell, or a CHO cell.

20. A method for producing a foreign gene-derived protein, comprising culturing the transfected cell according to any one of claims 17 to 19, and obtaining the foreign gene-derived protein from the culture.

21. Use of the polynucleotide according to any one of claims 1 to 10 for the purpose of expressing a foreign gene in a transfected cell.

22. Use of the foreign gene expression vector according to claim 15 or 16 for the purpose of expressing a foreign gene in a transfected cell.


SEQUENCE LISTING

<110>  DAIICHI SANKYO COMPANY, LIMITED

<120>  Promotor of Hspa8 Gene

<130>  DSPCT-FP1927

<150>  JP2018-149854
<151>  2018-08-09

<160>  20

<170>  PatentIn version 3.5

<210>  1
<211>  2945
<212>  DNA
<213>  Cricetulus griseus

<400>  1
caaggctgag gcagcgccat ctggtactcc ctgcccccct ttctctcttg ataactgaga      60

ttataggttg gctccacagc tgccttggct ggaatttcca tctgataaaa tgaagtctgg     120

agccactagg attgcaccag gggcttagta aaagctcccc cacccccacc ccaatgaatg     180

gggcttctag tgagattcct ggaaagggag ggcactaagc agttggtaat ttagctggaa     240

atcttgtttt tcctcaggca caaggcttca tctggattcc taggtgagga gggcttcttc     300

gctgttctcc cctctctccc acactcagaa tttcctttct tcctatctgg ccctgctttt     360

ctttcttatt ttgagacggg gtttcactaa gttgtcaggc tagcctttac tcacttactc     420

tggagtagct agggtcgaaa gcactctcca cccacccgat ctcccagaaa gcacacttct     480

caaacagact ccggctcttc atttttaaac ttaaatttac aaattggttg acagtctcat     540

attccaacct ggtctagcag aactttaggt cctccacttc ctgcgtgccg actataaagt     600

gtgagccacc actcccagtt acacagtagc ttagaaaaag aggacaggat tgagtccaag     660

ataaacgttg tcatgtcttt gtgccttacg tttctaatat aggggtgagt attagaaata     720

cggttaagaa gtatgcactt tgaatgtaaa gtgcttttta catttacata aaaagcattg     780

tattaaaatg atcttcttgg acagtaacaa aaagtacttc cctccaggaa ctccaagcca     840

acattcttcc caagatacaa atacagaaga aaaaaaaata ttcccttatt agtgtctcag     900

taattactgg ctatgatgag gacaaagcct ccccagtctt ctgatagcct ttctcgcgtg     960

gtatggagaa tttccaatca aacaccgtaa gtgaaagttg ctacggtatt cttttaaaaa    1020

acaacctaac taatagctgt cctgcccttt tgaaaggaca cgtggaaagt gctggagaga    1080

cgggcttaat gacaaggccc caaacgaacg ccctgcctcc atgccttgca gtttcatagt    1140

ccaagcactg tcacgttttc aaggactctg cgcggtcgag cgtttaattt ttggtataag    1200

tcaagtcagg ggagacatta cctcatgtta ctgaaaataa aaggtcgaga tcgccgcatc    1260

aaggacacag ggctgccgga aacgaggatt gccaaggcct agctcactct cagggggaaa 1320

aaaatatacc cttttgcaca ttttttttct tttttcacaa agtgagaggt ctggtggggc 1380

agggagggga cggaggccac gctatcctag cccgggggtg gggaggggcg ctcggctctg 1440

gggtcggcca agaagacgag gccatttctt tcactgaggg tcacaaccga gcatcctggg 1500

gacctgtaca agtcaggcac gtctaacctc cgcactgggg ggctgcggac tgacgtgttc 1560

catttctcag agacaccgcg cggcggccga gccatctcca aacgcggcgc cagacccagc 1620

tatcctcgcg gtccccacta gtctcagact cagacaccgg gggcgcgcga agcctcaggc 1680

cgctggcccg gcagcccgac aggctgaggg agaaggaccc aggacggggt gggggagggg 1740

cgcaacaacc ttcgcggcca ttttgtcctc gccccacttc cgtcttctct tcccggactc 1800

cccctccctc aggctcttct ctccggcggt ccggggtccc gagaggcccc gaacgcggag 1860

aacactgctc tcattggtcc gctgtgaatc cacccgcctc ccttcgtcgg cttccttccg 1920

ctccctcccg cacgcgctga ctgggcgcgc atgcgtacag gctgccgagt ccctcccctc 1980

ccccgccagc cgtgagtgac agaggactcc cgcgcgcggg ctctcggcgc cccgccctcc 2040

cctttccccc gggccgttcg gagagcgggg gcgagcatga aagttccaga acgctgcggt 2100

cgtgcgtcat cgccaggtgg ggcggggcga gggcgggacc taggagagcg tctcgggcgt 2160

tcattggtgc tgttcgaaaa gccccatcgt ccattggcta aggagccatg ataggcagcg 2220

ggacggggcg ggatccttct ggaaggttct gagacagggt ataagagaca gtgaggcggg 2280

cggaaaccgg tctcattgaa cgcggaggca gcttgtgagg ttttgtgtgg tctcgtcgcc 2340

agcacagccg ggcctacagg caaggtgagg cctggggctt gggtggtggc gggtggcctg 2400

ctttactgca gagcggcggg gcactgaggc ggcgggcggg ggcttaggcc gcggcctccg 2460

gggacccggg ctgcagctga ggaggatggg cttaggtagg gcggcgccgc catggccttg 2520

aggctcgtcg cggcggggga ggggtgccgc agccgcccgc gccgctccct ttcacccgtc 2580

tgcggattaa acgagagggt ttctgaccgc ggcccaaggg cgagctgctt ggaatatttc 2640

agcaggcgag tctggcgccg caatgcgcgg tcctggcggc tggaaggggg gaggggttgg 2700

gttctccgag ttgaggccac gtgctcaacc attgtcgcag accgtcagga cagatggttc 2760

ttcagccagg ggcgggctct gccagggttg gcacggctac cggactgcag cagtgccgcc 2820

ctgactgagc cttttgcgtg gcctcaggtg ataggtctgg gctgatgagg acatacggat 2880

tgcgggtctg gaagctgggg aatagctgct tagccgtttt cgtgtttggt tttctttcag 2940

caacc 2945

<210> 2
<211> 2000
<212> DNA
<213> Homo sapiens

<400>  2
atacaaacgt tcagaaagtc taaatcaagg acgcacggct actggaacct atgattccta      60

atgcctggtt cacctaacca agaacacact cgccaccatt taaacctgtt tattttataa     120

acaacgggga aatgtagggc ttgggctagt tggggaagga ctgagccaag aagccgaatc     180

tgttctagac ggaggtggga aggggcctaa cggtcagggc tcagagaagc ggtttcgcct     240

tcttttccgc ggagggatcc agacacgacc gcgtcctgag gcggttctga aaagccgggc     300

cccttaaacc tctgcgtcgg gggctgcctc ccgacagttg ccgtagggaa atgcaggtgc     360

tgggaggcct gccgagctaa cccgccccac cccgcggcgg cctggcggct ccctccaatc     420

ccaatcctgg ggggccgtga gcgagcagcc ctagtggcac cctgaagccg gaaccttcgc     480

cccagcccct cggggtcagc ccttgtccaa agccacgcta tgaaatacag attcgcgaaa     540

cccggggcac ttagaggccc gtctggaaca cgaaggtggg gcatggaaag gaggattcgg     600

acgtgggtgg gggaggggag caacaacctt cgcagccatt ttgtcctcgc tccacttccg     660

tcttctcctg cccggctccc gcgcccaaac ccctcccttc aggccccgcg cgattccgcc     720

cccagttctg tgccggccaa gatcccggct agcgccgcta tcattggtta gttccaagtt     780

tgcccgcccc tcttcctcct cctttttccg ccccctccct cccgcggaag ctgggggcgc     840

atgcgtagag gtggacgctc ccctcccccg cccggggtaa ctgaggactc ccgcgcgcgg     900

actcgctgcg ccccaccctc cctttccccg gggccgtccg gagagcgggg gcgagcttga     960

aagttccaga acgctgcggt gagtgcgtta tcgtgaggcg gagcgcggtg gggtgggtgc    1020

ggaagggggc gaggcccgag gagtggagcc gggcttgtga ttgggtcttg taagggcagc    1080

cgggcgtcta ttggccgggg aagccgtaat ggcaggcagc aggggcgggc cccttctgga    1140

aggttctaag atagggtata agaggcaggg tggcgggcgg aaaccggtct cattgaactc    1200

gcctgcagct cttgggtttt ttgtggcttc cttcgttatt ggagccaggc ctacacccca    1260

ggtaaaacct ctgctcaaga gttgggttgt gggtctggga gcgtgcagcc tccacacagg    1320

cctgttgggc ttgctgaggc ttgggggttc tgagaatctc gtcgaggcga gtgtgcggct    1380

ccttctaccg gcttaaaggg cctcagtttt cggtgggatg gcagcggtat ttggttgcag    1440

ccggcaggac ggaaatgtag ggagtgggcc gcagtggccc caggggaggc tgggagacgc    1500

ccggcggccg cgtggcgggg gagggttgct gcatcggttt gcctggcgcg cggggaagtg    1560

gagccagcgt tttctttcac ccagttccct gcttagtcca gtcccaccgt ggttcttcag    1620

agctgttctt ggcgtgcttc cagtatgggg gtacattccg gagtagttaa aagcccgttg    1680

actcccgggg gcactggcac ctggcgaggg aggggaacag acagtgctca gttcggggta    1740

agaccacgtg ttgagcaacg ccccacgccg tctgggtaga tgggtccttc atctagggcg    1800

tgctctgctg cggttggcac ggcaacctgg actgcagcac tagttctgga cctcgcgcgt    1860

gcttagacag gaggtgatgg gcactattac ctcttggcag tggccatacg tttttcctgg 1920

ttaagtgttc tgttaaggga tgagggaaat attttgatta attgaatttt taaaccagat 1980

ttttcttttt ttcagcaacc 2000

<210> 3
<211> 2000
<212> DNA
<213> Mus musculus

<400> 3
   agaccttcca atttaaacgc cacaacagta ttctttaaaa aataaaaaga caacctatct 60
   aactaataaa gagctgcact ccctgcctat ttgaaaggac gcgtggaaag tgctagagag 120
   ccgggtgtga cgaggcccgg aacgcccagc ctccaggcac tgcggtttca gagtctaagc 180
   actcgctccg gccattggct cagtcacgtt ttcaaggact cagcgcggcc gggcgtttaa 240
   tttttggtaa aagtcaagtc aggagagaca ttacctcatg ttactgaaaa taaaaaggtc 300
   gcgatcactc tatcaaggac acggggctgc cggaaaccag gaatgccagt gcctagctca 360
   cccacaaagg gacaaaaaac atttatcttt cgtacattta ttccattaac tgatgagtct 420
   ggtggagcag agaggggaca gaggtcacgc tgtcccctgt gggacagcgg aggtggggag 480
   gggcgttcgg ctcaggggta agcagagagg actgggccat ttctttgata gaggatcaca 540
   aacgatcctg gggacatgtg gcgagccagg cagatcgagc ctccgcactg gggggggggg 600
   gaatcgaggt gtcccatttt gtggggggtg aggggtgggg aaagccagcg agatagcgcg 660
   gcggcttagc ctcccacaag ctggcagcac gaaacaagaa gcctagcagt gctccttagt 720
   ctcaggctgg ggtggaccaa gcccgaggcc gggaaaccgc cgggctcagg gagaaggacc 780
   gagggtcgtg tgggggaggg gcgcaacaac cttcgcagcc attttgtcct cgccccactt 840
   ccgtcttctc ttcccggact ccccctccct caggctcttc tctcaggcag cccggggtcc 900
   caagaggtag ggatccggcc gaacgctgct ctcattggtc cgtcgcggag gtacccgcct 960
   tcctcccagg gcatccttcc gccccctccc gcctgcgctg ccgggtcgcg cacgcgcact 1020
   cgccggcgac cctctcccct cccccagcag ctctgagtga cagaggactc ccgcgcgcgg 1080
   gctcccggcg ccccgccttc tccgctcccc ccgggccgtt cccagagcgg gggcgagcgt 1140
   gaaagttcca gaacgccgcg gtcgtgcgtc atcgcgaggc ggggcgggga gaaggcggga 1200
   cctaggcgag cgttctggct cttcattagt gcagtcggca agggctggtc gtccattggc 1260
   taagaagcta tgatcggcag cgggatgggg cggcaccctt ctggaaggtt ctaagacagg 1320
   gtataagaga gagggaagcg ggcggaaacc ggtctcattg aacgcggagg cagctgcctg 1380
   gcatttgtgt ggtctcgtcg tcagcgcagc tgggcctaca cacaaggtga ggcatgggac 1440
   tcgggggttt gcgggggtcc ggggtggtgc aggagtcccg gggagtctct taggcggcgg 1500
   ctgcagctca ggccgtcacc accagggaca gagactgcag cagatccgga ggatgggctg 1560
   agtcagggcg gcgccgccat ggccttgtgg ctccgcgtgg cgggggaggg gagccacagg 1620
   cgccctggtc tttttatcct gtctgtgact tcaaatataa ggcttctgcc cggcgcccac 1680
   agggcagggc cccccgaggt gggaaagggg gatcctggct cccgcggtgc gcggtccggg 1740
   tggggggggt tcggggggcg ctcggaggtg gccacgtgct ccaccattgt cgcagacctt 1800
   taggacagat ggttccttga gccttgggcg ggctgtgcca ggcttggcac agaccaccta 1860
   tactgcagca ctgctgccac gagcctgccg cgtggcttca ggtgataggt ctgggtcacc 1920
   aagtccagac ccactgcgga ctggataaaa gccggaaagc gagtgcttag cccccctcct 1980
   ttttttttct aatagcaacc 2000

<210> 4
<211> 2000
<212> DNA
<213> Rattus norvegicus

<400> 4
   cttttgatag ccttcctcac atggtctgga gaccttccaa ttcaaacgct acaactaaaa 60
   gttgttgcag tatatatatt taaaaaaaaa gaaaaaaacc aacctatcta actaataaat 120
   atctgttctc cctgcccatt tgaaaggaca cgtggaaagt gctagagaca ggcttggtgt 180
   gacaaggccc ggaacgccct gcctccatgc actgcggttt cctagtccga gcatcggctc 240
   tggccactgg ctcagtcacg ttttcaagga ctctgcgcag tcgggcgttt aatttttggt 300
   aaaagtcaag tcaggagaga cattacctca tgttgctgaa aataaaaggt cgagatcact 360
   caatcaagga cacagggctg ccggaaacca ggattgccaa tgcctagctc acccacaagg 420
   ggaaataaat tatccttttg aacatttatt ccataaagtg ataagtccag tggggcagag 480
   agggaataga ggccacgctg tcctcgtcca gagatgggga ggggcgctcg gctccggggg 540
   tcagcaaaga ggactggacc atttctttca cagaggatta ccaacgagga ccctggggac 600
   atgtgacaag tcaggcatta tcgagccccc acactggggg atcgacatgt tccattttgc 660
   tggggggaca gcaagggagg tcgctcggcg gcctagcctt ccacaagaga ggctccacga 720
   aacaagccta gcagtgctcc ttagtctccg gctcggaagc cgtgggtgga cgaaaccgga 780
   ggccgggaaa ctgctggact cacggagaag gaccgagggg tggggtgggg gaggggtgca 840
   acaaccttcg cagccatttt gtcctcgccc cacttccgtc ttctcttccc ggactccccc 900
   tccctcaggc tcttctttca ggctgcacag ggtcccaaga ggaacagata gcgccgagca 960
   ctgctgccat tggtccgttg cgggtgtacc cgccctcctt catgggcatc cttccgctcc 1020
   ctcccgcctg cgctgacggg tcacgcatgc gtactcggtg ccgacgctct cccttccccc 1080
   accagctctg agtgacagag gactcccgcg cgcgggctct cggcgccccg ccctcccctt 1140
   tcccccgggc cgttccgaga gcgggggcga gcgtgaaagt tccagaacgc tgcggtcgtg 1200
   cgtcatcgcc cggaggggcg gagtggaggc gggtcctagg agagcgttct ggttgttcat 1260
   tagtgcactc cggagaacct ggccgtccat tggctaagga gctctgatcg gcagcgggat 1320
   ggggcggaac ccttctggaa ggttctaaga cagggtataa gagacagtga agcgggcgga 1380
   aaccggtctc attgaacgcg gagccagctc tcgggggtct ctgtgtggtc tcgtcatcag 1440
   cacagctggg cctacacgca aggtgaggca tgggacttgg gtgttggtgc gtggccggtg 1500
   tggtgcaggg gtcagtggga tgcttaggcg gcgggtgcgg cttaggccgc tgttgccaga 1560
   gacggaggct gctgctgaag gggaggatga gctgagtcag ggcggcgccg ccatggcctt 1620
   aagcctccgc atggcggggg aggggagctc aggattccgg ggccttttct tttttctgtc 1680
   tgcaagctaa aggagggtct cttccgcgcg ttcccagggc acgggtttca gagatggaag 1740
   aggcgatcca gcctcccgca gtgcgcggtc ctgggggtgg ggggggccct cggaggtggc 1800
   cacgtgctca gccattgtcg cagacctttt ggacagatgg ttccttcagc ctagggcggg 1860
   ctgtgccaag cctggcgcag gccactcaaa ctgcagcagt gtcgcctgcg tggcttcagg 1920
   tgattgatag gtctgggccg gtgcagagtg aaagcaggga gatgaccgct tagtcctttt 1980
   tttttttcct tctagcaacc 2000

<210> 5
<211> 1926
<212> DNA
<213> Cricetulus griseus

<400> 5
aacaacctaa ctaatagctg tcctgccctt ttgaaaggac acgtggaaag tgctggagag 60

acgggcttaa tgacaaggcc ccaaacgaac gccctgcctc catgccttgc agtttcatag 120

tccaagcact gtcacgtttt caaggactct gcgcggtcga gcgtttaatt tttggtataa 180

gtcaagtcag gggagacatt acctcatgtt actgaaaata aaaggtcgag atcgccgcat 240

caaggacaca gggctgccgg aaacgaggat tgccaaggcc tagctcactc tcagggggaa 300

aaaaatatac ccttttgcac attttttttc ttttttcaca aagtgagagg tctggtgggg 360

cagggagggg acggaggcca cgctatccta gcccgggggt ggggaggggc gctcggctct 420

ggggtcggcc aagaagacga ggccatttct ttcactgagg gtcacaaccg agcatcctgg 480

ggacctgtac aagtcaggca cgtctaacct ccgcactggg gggctgcgga ctgacgtgtt 540

ccatttctca gagacaccgc gcggcggccg agccatctcc aaacgcggcg ccagacccag 600

ctatcctcgc ggtccccact agtctcagac tcagacaccg ggggcgcgcg aagcctcagg 660

ccgctggccc ggcagcccga caggctgagg gagaaggacc caggacgggg tgggggaggg 720

gcgcaacaac cttcgcggcc attttgtcct cgccccactt ccgtcttctc ttcccggact 780

ccccctccct caggctcttc tctccggcgg tccggggtcc cgagaggccc cgaacgcgga 840

gaacactgct ctcattggtc cgctgtgaat ccacccgcct cccttcgtcg gcttccttcc 900

gctccctccc gcacgcgctg actgggcgcg catgcgtaca ggctgccgag tccctcccct 960

cccccgccag ccgtgagtga cagaggactc ccgcgcgcgg gctctcggcg ccccgccctc 1020

ccctttcccc cgggccgttc ggagagcggg ggcgagcatg aaagttccag aacgctgcgg 1080

tcgtgcgtca tcgccaggtg gggcggggcg agggcgggac ctaggagagc gtctcgggcg 1140

ttcattggtg ctgttcgaaa agccccatcg tccattggct aaggagccat gataggcagc 1200

gggacggggc gggatccttc tggaaggttc tgagacaggg tataagagac agtgaggcgg 1260

gcggaaaccg gtctcattga acgcggaggc agcttgtgag gttttgtgtg gtctcgtcgc 1320

cagcacagcc gggcctacag gcaaggtgag gcctggggct tgggtggtgg cgggtggcct 1380

gctttactgc agagcggcgg ggcactgagg cggcgggcgg gggcttaggc cgcggcctcc 1440

ggggacccgg gctgcagctg aggaggatgg gcttaggtag ggcggcgccg ccatggcctt 1500

gaggctcgtc gcggcggggg aggggtgccg cagccgcccg cgccgctccc tttcacccgt 1560

ctgcggatta aacgagaggg tttctgaccg cggcccaagg gcgagctgct tggaatattt 1620

cagcaggcga gtctggcgcc gcaatgcgcg gtcctggcgg ctggaagggg ggaggggttg 1680

ggttctccga gttgaggcca cgtgctcaac cattgtcgca gaccgtcagg acagatggtt 1740

cttcagccag gggcgggctc tgccagggtt ggcacggcta ccggactgca gcagtgccgc 1800

cctgactgag ccttttgcgt ggcctcaggt gataggtctg ggctgatgag gacatacgga 1860

ttgcgggtct ggaagctggg gaatagctgc ttagccgttt tcgtgtttgg ttttctttca 1920

gcaacc 1926

<210> 6
<211> 1199
<212> DNA
<213> Cricetulus griseus

<400> 6
aaccttcgcg gccattttgt cctcgcccca cttccgtctt ctcttcccgg actccccctc 60

cctcaggctc ttctctccgg cggtccgggg tcccgagagg ccccgaacgc ggagaacact 120

gctctcattg gtccgctgtg aatccacccg cctcccttcg tcggcttcct tccgctccct 180

cccgcacgcg ctgactgggc gcgcatgcgt acaggctgcc gagtccctcc cctcccccgc 240

cagccgtgag tgacagagga ctcccgcgcg cgggctctcg gcgccccgcc ctcccctttc 300

ccccgggccg ttcggagagc gggggcgagc atgaaagttc cagaacgctg cggtcgtgcg 360

tcatcgccag gtgggcgggg gcgagggcgg gacctaggag agcgtctcgg gcgttcattg 420

gtgctgttcg aaaagcccca tcgtccattg gctaaggagc catgataggc agcgggacgg 480

ggcgggatcc ttctggaagg ttctgagaca gggtataaga gacagtgagg cgggcggaaa 540

ccggtctcat tgaacgcgga ggcagcttgt gaggttttgt gtggtctcgt cgccagcaca 600

gccgggccta caggcaaggt gaggcctggg gcttgggtgg tggcgggtgg cctgctttac 660

tgcagagcgg cggggcactg aggcggcggg cgggggctta ggccgcggcc tccggggacc 720

cgggctgcag ctgaggagga tgggcttagg tagggcggcg ccgccatggc cttgaggctc 780

gtcgcggcgg gggaggggtg ccgcagccgc ccgcgccgct ccctttcacc cgtctgcgga 840

ttaaacgaga gggtttctga ccgcggccca agggcgagct gcttggaata tttcagcagg 900

cgagtctggc gccgcaatgc gcggtcctgg cggctggaag gggggagggg ttgggttctc 960

cgagttgagg ccacgtgctc aaccattgtc gcagaccgtc aggacagatg gttcttcagc 1020

caggggcggg ctctgccagg gttggcacgg ctaccggact gcagcagtgc cgccctgact 1080

gagccttttg cgtggcctca ggtgataggt ctgggctgat gaggacatac ggattgcggg 1140

tctggaagct ggggaatagc tgcttagccg ttttcgtgtt tggttttctt tcagcaacc 1199

<210> 7
<211> 8450
<212> DNA
<213> Homo sapiens

<400> 7
attttgcttg aaaggatagc atcaaggaag tgaaatgaca acccacagaa tgagagataa 60

tttttgcaaa tcatgtatct gataagggac ctgtagtcag aatatgcaaa gaacccttac 120

aattcaataa gacaacccaa tttaaaaaca ggcaaaggat gtgaataggc atttctccaa 180

agatacggaa aaacggccaa taagcacata aaaagatgct caaaatcatt tgccatttgg 240

gaaatgcaat caaaaccaca atgaggtatc acttcacgcc cattagggtg gctatagatc 300

agaaagtcag ataacatgtg ttggcaagca catggaaaca ctgaagtcct tacacactgc 360

tggtaggaat gtaaaatggt gcagccactg tggaaaacag ttttccaatt tctcaaaatg 420

ttaaacacag ttatcataca cccaagcaat tctactctta ggtatatacc caagagaaat 480

gaaaacatat gtcttcacca gaacttgctg ttcacagcag cattatgcat aatagaccaa 540

aagtggaaac aactcaactg cccatcaact ggtgaatgga taagtaaaat gtgatgtaac 600

cagtcattgg actgtcattc attaataaaa agaacaaggt actgattcat gttctaacat 660

gagtgaatct tgaaaacact atgctaaatt aaagaagcca gtcacaaaag gccgtgtatt 720

gcatgatttt atatatacat gaacttttat atatatataa ttatatatat tatatataat 780

tttatatata taaatttcta tatataaata tataaaatca tatatatgat atatattttt 840

tcatatacat catatatatt tacaaaaatt atatatcata tatcatatga tatatgagat 900

atatatcatg atatatatga tatatgatat atatcatatg agatatatga tatcatgaga 960

tatatgatat catatgatat atatgatata gatatcatat gatatatata taatatatat 1020

atgatagata tattatatat gatagatatg atagatatca tattatatat gatagatatg 1080

atagatatca tattatatat gatagatata gatatcatat tatatatgat agatatgata 1140

gatatcatat tatatatgat agatatgata gatatcatat tatatatgat agatatgata 1200

gatatcatat tatatatgat agatatgata gatatcatat tatatatgat agatatgata 1260

gatatcatat tatatatgat agatatgata gatatcatat tatatatgat agatatgata 1320

gatatcatat tatatatgat agatatgata gatatcatat tatatatgat agatatgata 1380

gatatcatat tatatatgat agatatgata gatatcatat tatatatgat agatatgata 1440

gatatcatat tatatatgat atcatatata taccacatac atcatatata catcatatat 1500

acatcatata tatcatacat atatatgaac tttccagaat aggtatatca ataaagacag 1560

gaagtataca agtggttgcc acagcctgag aggagcaggg aatggtgagt gactgctaat 1620

ggatatggca ctttttttgg ggggtgatga aaatgttctg gtcagacaat ggcaattaca 1680

aaactgtata cacacgaaaa accaaagaat cacacacttt aaaagggagg atttagctcg 1740

gcatggtggc atgcgcctgt actcccagtt actcgggagg ctgaagcagg actgcttaga 1800

gcccaggact tcaaggctgc agcgagctat gatcgctcca ctgcactcca acaaggatga 1860

cagtgcgaga cccgttttct aaataataat aataataata ataataaata acccaaggta 1920

cccagttcac atgcaaaacc actggtaaac ataaattatc tccaagtaat ctagaaagaa 1980

aatgagcaca taagacgtct tctaaaaaca cacatatatt tctttacatg ttacatttaa 2040

cgtaaaaatc agctatgcag aagttacatg aacattttat gttggaaagg taaatgacta 2100

ttattaatac agaatggtta agtacattta tgtttttatg tacaaacgca taaaaggaaa 2160

agcatcctta aaataaacac catcaatggc tcctcggtgg tcacaaaaca aaatcctcac 2220

acctttgtct tccttcacaa ttgagcttta tccacctttt caggcttatc tcccattatt 2280

acctgacaca aacttgggtg ggccagagtt tccactgacc atcccccgac tattcatcca 2340

acactatgtt cactgcctcc cattcctgac catttgcctt ttgtcttcaa ctaattctgg 2400

ggacgttttg tccaaataaa tgatccatat tcttgaaggc tggaatcaag tcctattaca 2460

aatatatttt ctcaccctct ccagagcata gcaacccagc atctactggc ctctcacagc 2520

tctaaccatc cacaacccta agctggcttc tcatcaaacg ggtacttttc accacccaaa 2580

ttcaattaat tcactcttac aataatgaag aatagtcgcc tacagcctac cttttccagc 2640

cttgattcaa tcatttatca attttatctt caaagtccct cacttcaggg agatgatata 2700

tcagctttca cccagagtcc taaagaaaac agcactcttg ccaatgacat agtgccacct 2760

agtggcaaca taaggtaaat cacagtggca gtagaaggat ctccacacta cttttacagg 2820

aatgcactgc aggtaaaaaa taagaagcta cagtactgtt tggcaggaca atttgtttca 2880

tacgtgcata ctatcgccct gactaaatta actcgcaagt cttacaggta ttatttgttt 2940

tcagttccat gcacagatta gccatttagt acttactaaa tcaaactcaa tttctgaagt 3000

gtcttacacc aatatattca tgcacatatg gttaaaattt tccttgagga tctatcatgt 3060

gagagtgtgg cttattataa caagtaaaca gaacaaataa atacaaaatg aaaagaaatc 3120

gtatgattta ctcgcatata agggagcttg ttgtggatta agtttcatga cccaggacac 3180

tgaaacagaa atggaataaa tgagaataaa attaaaagtt gtcatcaaaa atatagaagc 3240

catctaaaga cctaggtgtc aagcatagct ctatgagtac aatcccgtgc ctgagattac 3300

catatgccca gctgtatgct atacactaag agatttagga aggaagcggg gtcagggatt 3360

gaccccagac tccatctttt caagtgggga agaaagatct tccgattgaa aaataaaggc 3420

aaaaaaggct tcaccgtcac agaagtttca acaaccaaca ggatatttaa aacagttatc 3480

aaagcaaaac cattgtatgt tcacttacat ttttacatag tccctcaaac tcacaaaatg 3540

ctgtttactc agggacttct tccggtctta ctagggagcc tggaaagtga cgggaggatt 3600

gcaagggacc actagaaccc tcttcctcaa ttccccttct ctgagaaggg aggctacagc 3660

ttgcctctct aaccactaaa aggcatgacc ctcctcaaag ttaatagccg gattccctga 3720

tagatatttt cactaaatga attctcataa aactctcact aagatttaga gaaggcttcc 3780

agggttgaat tcctgaacat taagaacagc atgtttttta aaagtttaac ttggtgattg 3840

gaccaggact tcatctaggc tatgaatgct cagaatggta ggtcctttac caaacagctt 3900

gagtttgtgt ataaagtgat ctcatcctct taagagtcag agaaacagaa ccaagcgact 3960

tcactataat ttgatctgag gaagtttctt actcacaata ggtaaatgaa ggcacatact 4020

aaccagcaat ataaacaaca atatcaagtg tcattcacac atgcaaaaaa cagacaaaat 4080

cccaaactct gtgttctaac aaatcgcaaa aacctcacta acaataaatt gaaatgacca 4140

aatgtttgga ctgaaaagca atgccttggt agcctagcca tgcctaactc aaataacaga 4200

accatctcga tgttaaaatc ctcacagatc aagctgtgta tgtctcgggt caagacttcg 4260

ccaaaaagca gtgagcacac acttaagagg gaaaaaatct acctcagcct cctaaatgca 4320

atcatctcta cacgagttgc aggccccaag cttcaacgtg ttctgctgga caacgcagta 4380

gaaagctgac aagcaggtgg ccttcccaca ctgactgaac cacctccatg cccatgtcca 4440

ttcattttct tgcccacccc atgtgctata acagacctcc tggctcaggg cactctttcc 4500

ttcctgactg ccttcactta atgactttgt acttttaggt gcaaaaatta tctgcagaaa 4560

tccacactga aaaccaagct tgagaaaggc agcaataacc aacattttta caagaagaac 4620

aaggtcaata tcaagcccat cagattcaaa tagcaagcat ggatgaaaat gaaagattga 4680

aaggcttgag tgccttctta atgtattaaa tatccattta atttacaatt aagctcactg 4740

tgctcactgg ccttttaatc agctttccag gtcctgctca gacttgccta ggacatggga 4800

atgaaagaac ctatacattt atggaccaat ctaccttaac taacttgtca agtgttcctg 4860

catcaagcag aagaaacatc agtgaaactg atacaggaat taaccccttg ttaatccata 4920

aaacttaaag gagcgggatc caatcttctg gcttccctgg gccacgctgg aagaagaatt 4980

gtcttgcgcc acacataaaa tacacgaaca ctaataatag ctgctaagct ttaaaaaaat 5040

tgcaaaaaag gaaaatctca taattttttg tttgttgtga ggtggagcct cactctgtca 5100

cccaggccgg agtgcagtgg caccatcttg gctcactgca acctctgcct cctgggttca 5160

agccattctc ctgcctcagc ctcccgagta gctgggatga taggcgtgtg ccaccatgcc 5220

cagctaattt tcgtattttt agtagagacg gggtttcacc atgttggcca ggctggtctc 5280

aaactcctga cctcaggtga tccacccacc tcggcctccc aaagtgctgg gattacaggt 5340

gtgagccacc gtgcccggcc aatgttttaa gaacgtttac gaatttgtat tgggccacat 5400

tcaaagcctt cacaggctgc atgcagcctg caggccgcgg ttggacaagc ttggattaga 5460

gaaatctaca gagacaaact agtgacttag tagccctctg atagctcatg atttgcaaga 5520

aacttaggat gactatgtgt aaagaccaca aacatcaatt taactgaatg gttcccgcca 5580

cactggaatg aggaagctga gcaaactcag aggactctaa gaaagggctg atgtcatctg 5640

aactgttcgg aattataaac tcctctaaac atgtttcaaa gccagaactt gtaggagttg 5700

ttctgataca cggattaaaa gagggatgac aaagtgtctg tcccccacac tggtcaaagg 5760

gacaggtcat tgttatgctg gcaatgcagg ctgctgaaaa gaatgtatct gtcaaaagta 5820

atcaaagtaa tgaccccaga aggctccaga aacagactgg taaattcagg ttgctttcag 5880

acttccacaa tgctggcaca caaggggaaa gacaaaacta acatttacag agcattatat 5940

ttgatattac atttaatccc cattaaaaag atactatttc ccgtttcact agtgaaaaag 6000

ttgatctttc aaaggttaaa ttatttaaca ccaaggtcaa agggtaagtt ggagagacca 6060

gattcaaacc cagtctgaca ttaaaacatg tgttttcccc ccacatcgtc tcctgctaat 6120

aacctcaaat ctaaaaactg acttgcccta caccttgagc cccatcctac aaactctccc 6180

tgacgttatt aattcagctg tcactgtgca cctacaacgt gccagacacc atactcctca 6240

acactctgta ggcacagaag gaacagataa aaatccctac cttcatagat attattctag 6300

gggtaacaca ggtaaataaa acattaaaat agttttcaca tagtagcaaa ttccatatag 6360

caaaataaaa cagaagaagg aatagcaaat gagggagatg ccctcttaaa catggtgctg 6420

agggaaggcc tccctgagaa agatatcatt taccccaaaa ataaaaaagc aagtaataga 6480

aaaaacaggt aaaaggtgtt ctagacactt aaacctgcca cattgagaac tcagggttct 6540

gatgcaaaac ctcgctgcat agaatgcatt aacttatttt tatacattta aacaaacaaa 6600

ctctacttaa gaactgtgtt ctaaaggaag gagcatatta caggaaggca atttttggtc 6660

agagtagaca cacttaaaaa ctaaacctat tgaaagacca agaacaactg aaagtctttg 6720

ctttgtcaga tttttgacca aaaggaaaat taaagaaaca caccgtgccc atccaatgat 6780

ttcaccaagg aattttaaga gagaaaatcc tacttcttcc tcacccagta gccagtgaaa 6840

tgactgagca aattcacaag ttcactgggg ctgctttcat gtaacacagg gacaacacat 6900

gacagacaca gtggaaccct acaggttgcc tagtatttga aagactgtga agaggaggag 6960

atgtcaaaat tcaaagtctt aaatgatgta gttttaagta tgttcagcaa tttcaccact 7020

cagtagtaaa gccagctaca gttgaaagca atcagaaatt tgaggggtgt gaaataagca 7080

gaagcacaga agttaaggat ttgtattctt cccacatttt ccactttatt ttatactgct 7140

gagaaaaaac aaatttaata gttttctgct gtataagaga gacacattca ctttatgtca 7200

cagtaagagt cactcaattt taatacaact atctcaatgt ataaattaac attctccccc 7260

ctgcccacac atagtaagtc tcttatgatg ttgctgatta gagaagcaaa agttgccgct 7320

acaattctct tcctgcattt taatataaac aatcatcagt cttttcttca tagagtgcag 7380

tgtgggcact atcatcagaa tgtaccagca ctgggtgtgc aaagtttaca aagattagca 7440

agagcaaaag tgttgagatt tttgaaattc atgctgctgc aaagaagtat gtaaaaactc 7500

actcaccata gaggaccaca cagaaactca ggcatgaagt tatatggctg tgtgagtggt 7560

ttgggagaag gaacggaaag cacttccacc aacctatatg cctgagcaaa ttaatgcaaa 7620

acctcagaag ctacaaaaaa gtttatctac ctaaattaaa attggtgtcc acagcagtag 7680

ccagcaaaat gcctgcgaag cgcaaagtgg taaatatttt agggtctgta ggtcatatgg 7740

tctctgttaa acaatatgta aatgaatggg tgtggctgtg ttccaataaa acttcattta 7800

taaaaagagg cagcatggta catccagtca gcaagctata atgtaccaac ccccggtcta 7860

acactaacca aatacctctt aataagccaa agaaactgtg tcctcttagg ccggaagcgg 7920

tggctcacac ctataatccc agcattttgg gaggccgagg cggggagatc acctgaggtc 7980

aggagtttga gaccatcctg gccaacatgg tgaaacccta tttctactaa aaatacaaaa 8040

attagccagg cgtgctggcg ggcgcctgta atgccaacta ctggggaggc tgaagcacga 8100

gaatcgcttg aacccaggag gcagaggttg cagcgagcct agatcacgcc attgcactcc 8160

agcctgggca acaagagaga aactccgtct caaaaaaaaa aaaggaaata aaagtataca 8220

aagtgaaaac aaagaaatta aactgccctt atttgccagt gacattactg tctatgcaca 8280

aaattccaaa aatctacaaa aaagcttcta gtactaaaaa tgagtttagc aaggttgtag 8340

aatccaaggt cagcatataa cataaaatca ccttcctata tactagcaat caccaactgg 8400

aaattgagaa gtatcattca caacagtacc acaaacatga aataaatgtg 8450

<210> 8
<211> 8420
<212> DNA
<213> Homo sapiens

<400> 8
tcttagtatg gtaaaccttt tgaagtagat tcaaatgaga atgggaagag agaaaaggga 60

gagaagcaac ataagaaatc tcttttaagg aattttatat agagagaaac agaggaatca 120

gttgatagtt ggaaattatt ttaaagaaaa tgggttattt taaagaaaaa aggtattaca 180

acatgtttgc actattgtgg gaataatcaa gttgagacag aaaattattt tttaaggaag 240

agtctaattg ctgaagtgaa agagaatgaa tgagaccctg tgcataagtg tgatcagata 300

ggagcatgta cagctcaagt aagaacagga agaaagagac aataaacatg tacagatagg 360

atgggctggt cgatgtggtg gtgaaaagac atgcgagtta ttactgatta cttctatttc 420

cccagtgaaa taggaagcca ggttcataaa ccaaaatgaa gaggagcgag gcagtattgg 480

aagttcagga aaagtaatag gtgtaaaaat atgtaaagta gaattaccag ggagtatgaa 540

gatacatttc caattaagga tgaagaattt aaagtgaggc cagccaatac ccctgctttg 600

cttcagctac atcagctgca taggttcagg cacagaatac atggaacatt gtatttaaat 660

agggcctgga ttttacaaaa gtaacacaat gaagaagaga gatgcaaggc tatttgaggg 720

tgtttgtggg agagattgta aaatattagc taagtaagaa ggggactgca aattttagtg 780

gtataaagga atgaggaaaa gtgtaaatac agtggggtca aagaatgttt ggagccaagg 840

cactagaggc aattagctga aaatgtaggt gattattggt gagtgacatg gtttaaatga 900

aaagtataga agggtacaat tatccatcat gaaaagttct agggtacaac taagatctga 960

gtagctgaag tagaatgaaa gtagaatgga cctttccata tccagccagg ttcagtgaca 1020

gaaggttagg aaacaaatta taaaccactt gagagaacat atcccctaag ttgtttttgc 1080

tatttttctt tcagcatata tttgttggaa tgccaactat gttcagttca attaatatgg 1140

gcttcttaaa taagggctcc agcactggat aatcctgcca tttattttga tacattccat 1200

cctgctgctc agatctattg gcatctacag gatgtctttt gagaagatgg gcattcacat 1260

ccctatgtcc tagcaaattt ccaactcaga aaaccacatt aggcttctct atatatcttc 1320

caactatttc aatggaaaat acaattctct gatttcttcc tatgatattt atcaaagaga 1380

atggtgcctg ccagttctag ggtgggggaa ctcaatacaa atcaccaacc tttagatgac 1440

accctgtctt caaagtgctt tcaaagtctg gcagaaaaaa agtacccagt ggctataaga 1500

ccacccagga gttcagtcat gcattctaag tagcagatca ctggaatgta attggctagt 1560

gagttcattt tactcttctc ttcttggtca catgttaccg cccttgtacc ctgcacgttc 1620

tctttcccag acttacaaag catgttctct tgaattcgtt ctctttttaa attcacacag 1680

tcttaatgat tcttctttca caagagtctt tcactcttac aattcagttc aagtcatcca 1740

catgcttatt atgagcaagg gtctgggact taggggaaaa gggaataaaa agatgaatga 1800

aatgtgatcc ctgcagtcca agagcttgct gtgaaaaagg aagtttggct tacattgcct 1860

ccctaatccc ttggctaggc cagaacagaa tattgtctaa aacctcctca cgtcagcagt 1920

cctctggggt ggtgactgga agtagaattt aaacaaaaat ataattgaca cataataatt 1980

gtgcatactt atagggtaca atctgatgtt tcgatatgtg tttaaatggg tgcattgtgt 2040

aatgatcaaa ttgaggtaat ttatccacca ccttgaagag agatttttca atattctcat 2100

tgcgaagaag caggaatttt tagcagacaa ctgagatgct tcttgttcac actaagtcat 2160

tctgacgatg gatttacata acttgttgtt ttttttgtgt gtgtgttttt gagacagagt 2220

cttactttgt cgactaggct gaagtgcagt ggcacaatct cggctcactg caacctccac 2280

ctcccgggtt caaacgattc tcctgcctca gcctcctgag tagctgggat tacaggtgca 2340

tgcaactagg cctggctaat ttttatattt ttaatacaga tgggatttca ccatgttggc 2400

cctgctggtg tcaaattcgt ggcctcaagt gatctaccag ctgcggcctc ccaaagtgca 2460

gggattacag gtgtgagaca ccaagcctgg tacatttaca tttcttatct ggatctttcc 2520

tttagtaagt gctaaggaat cctacttccc ccaatatttt ttcctatttc aatgttttag 2580

catgtatcat gttactactt tgcagacatt tgattttccc ctttgtttac tgtaaagtat 2640

atttttatag cctttgtaat agaagtattc taaaatctgc ctgcaaccta tctttctgac 2700

tctgcatttt agggaataat tctctgttgt ggaatgaaaa aaaaaacaga gcctgtggag 2760

tcagagatct catttcaaat tatagttatc cctaggaata aatctgagtg acaggtagta 2820

tagtataata ataagtataa agctatggtt aaggaaaact caacaacctt atctgtaaat 2880

tgggatgaca acagcctacg tcaaaaaaat gtgaaggtaa atgagataat gtaaggctga 2940

tacttagtaa gcaatttaaa aacacccaaa aaactattgc catgattact ctacttactc 3000

tatttctcta tgctccaggc aaatgaacta ctaatgaccc aggggtcctt ccccattctc 3060

ttcttcacaa ggaaatattc tctctctgtg tgctgtttat taaaatctac tgcccctttt 3120

agaagccttt ccagatcatc ccatggccaa gaacgatcgc tgcttcctct tctttacata 3180

cagatgtttt tctcctgctt gacaattatt tttgtgcaat tattttcctt ttgattgtgt 3240

ttttaatgtc ccccccaccc cacaattttc cagactgttt gctccacgag agaggagacc 3300

atcatctctg tgctcaccgt tgtatgacca gtatcctgag gagtggctgt tacataatta 3360

catcaggcac tcaataaaaa tttgatgaat aaacactgga ttttaaggca ggtatcatat 3420

cttacatagc atatcatatc ttacatttta tgtccctcac ataaatacca cagagtgaag 3480

tatatgacag ataaggtcat ttctcttgat aagtacatag tccagtctga aacagatatg 3540

ccaaaaaaaa acaaaactgg agtaaacaag atgaattgtt ttaatagagg cactgtatta 3600

gtttcctagg actgccagaa caaatcacct caaacttagt ggctgaaaac aacaaaaatt 3660

tattgtctca cagttataga tgttagaagt ataaaattaa ggtgtcagtg ggattggttc 3720

cttctggggg ctgtggaaga gaatctgtcc caagccttca cactgtaaag tacagtactg 3780

gagggatagg acttcaactt gctctatctc agatagagag gagccatttg ttgtgaattg 3840

agaagagggg tatgttgaat ccataataag cacataaaaa cttggctggt tcataggaga 3900

agtaacatgt ttccagctct agtaaaaaac aaattgaagt ggcctataaa aaggtacaga 3960

gtacgacaga atgaaaaata aatgaacaag aatacagaga ggatgtggta aattatcatg 4020

tttccctaat atgttattgg acactaaatg gtattagaat tatttatcaa taataattct 4080

aaactgttgc aattgaaaga atatattaag tggtgttata tgagaagtgc cagggcattc 4140

tcatttctgt ccaatgggag aaacattttc gtttgagacc tccgtgaata atacagtctt 4200

ttagttagga gagctgcatt ttgagtggtg caggcagaat ggcgatctct cacccacaca 4260

aacactaaga tagagagaga cagagacaga gacagagaca gcagagagag acagagaaag 4320

gaagtacagg tactcagata gagataagcc atttcttgac attaagaaat aaagtagaat 4380

ccattggagg gaaataaaac tgcctcagga acagagttaa ttcacataca catgcaggta 4440

aacacacact gcttgatact tactgtggac tttgaaaatt atgaatgtgt gtgtgtgtgt 4500

gtgtgtacat tcagccctcc atatccatgg attttgcatt cacagattca accaaccatg 4560

aattaaaaac atttggaaat aacaaacatt aaaatataac aatacaacaa taaaaataat 4620

acaaataaaa aatatagtgt aacaactgtt tacatagcat gtatgttgta ttaagtagta 4680

taaatctaga gattacttaa tgtataccag aggatgcata ggctatatgc aaatactatg 4740

ccactttaaa ctgataagaa cagatactaa acttcatctt agccaaaagt cagagaaaca 4800

atataactat gccattttac ataagggact tgagctgagc atcctcagat ttcagtatct 4860

ttggagttcc tggaaacaat tccttgtttt atatatatat atgtgtgtgt atatatatat 4920

atatatatac acacatatat atatatatat atatatgata gctactgagt gacaggtgat 4980

attataccat accacttgtc actcagtagc tgtatatgca tatgtatata tatacatata 5040

catatatgtg tgtatgtgta tgtgtgtgtg tgtgtgtgtg tgtgtgtatg ctgtctttcc 5100

tcggtatcac agggaattgg agatatatat attcttttca gtacaaaaaa aattgaacac 5160

agatgggtat ggtaccagaa cagaaggtaa agacacatga aaaaaatttg caacaacatg 5220

aatggaactg gagatcatta tttgaggaga aataatccag gcacagaaaa acaagcattt 5280

tattatttta ggtgaaagac aaacatttta ttttaggtga aataatccag gcacagaaag 5340

acaaacattg catgttctca tttatttgtg ggatgtaaaa atcaaaacaa tagaacgtat 5400

ggaggtagac agcagaagga tagttaccaa aggctgcaaa gggtagtgta ggctttgagg 5460

gtgaggtggg gatggttatt gggtacaaaa aatagttaga aagaataaat aatatctagt 5520

atttaatagc acaacaggtt gactatagtc aaaataacat aattgtacaa tttaaatatg 5580

aaattaaata tatatacaag actagaacac caagttgaat gactccagct tgcgaaaccc 5640

acattgatca ccatgcttgc cccaagggaa gctgtacaat gtctggctcg tccagaaccc 5700

catcatttat cactagcaat ctattgtcca taatcatgtt taaattaata gcattttaaa 5760

ggtacaaata ttttttaaaa aacaaataat tatttaattc gccttttaaa agctttttaa 5820

aaacgttttt aaaaactttt ttaaagtcct gaggactatt ttctttaaag tgctcagtta 5880

cagagctcca tatattgggc tatgatagcc ttacctgatt cttgccaaga atctagtgcc 5940

cagaaaatgc aaatacaaag taagcaactg aaaaataaac aaataagttg gaggtatgct 6000

acctgttgaa atatgaccta gcgcaaacac ctatgccact tgcttatgaa atcatatagg 6060

ttttcggtgt gcagttttga ctgaatgagg gagtttacgc tggaccacaa gggggcccct 6120

ctgtcaataa cgtactccat ttgtgtatta agtcaaaaat gaaatggaag agaaaagaaa 6180

catcgatgac cccaagtctc tttaattgaa tggaggtaaa agggaaacaa cgaatgagaa 6240

aagtactctg cccttttaag aatcttgcat tcacattcct gatgaagtta tttttcctcc 6300

tctcactgat tcccatttca ctctattaca tagcaccgtg ttccccagga gctcctgaat 6360

gaaggacatc actcagctgt gttaagtatc tggaacaata aatatactag tttcaatgtc 6420

taggctatgg gtattccttt ttactgaagg tatgacatat agctgcccag gcctgactaa 6480

attaatagta ataataatta ataatggcaa atttttattc tattaagtta cttggcttga 6540

cttgtagaaa tagcaacatt catctgaaat gccccctcct acacttatgt ctaaggacaa 6600

atcccacata caccacagat aacttcattt tacatgtttt attctgttac caaactaaat 6660

ttttatcata tagtctgttg ctcactgaac tcttcagtaa ttctcaacat accatgtaaa 6720

gcattaagca cagttccaac acagagcaaa tgagcaataa ctgttagtta ttataacatt 6780

attatgtgtt ttcagtgcat taaaccactg gtctgatacc tagcccaaca ttctattaaa 6840

ccacataatc cagttgaata atatatgata atataataaa atggcgataa gtgctaaata 6900

tccagataga aacacagatg gaatcagaca gctttcccaa gaaatagaga aaatagtaga 6960

taggcgatct aggcctaagc actctaagca gaagctaagt tatcacagga tatcttggca 7020

atctgtggca cgtgaaccct tttcttctgg agtctggaac tatgttgcaa ctctcacttt 7080

ctccctatct agagactcag tttgttccct tgtgattatc agcagttgag aaatccttag 7140

accttctgaa aggactactt tttaaattta tatatataat atttaaaata catatcttta 7200

tatataatat atatttaaat atataatatt taaattaata tatatttaaa tatataatat 7260

ttaaattaat atatatttaa ataaataaat ttatatttaa atatataata attaaaatat 7320

atttttaatg aacagagagt aaaggattat tttgaagaga aactcctggt tcccacttaa 7380

aatcctttct tgtttccaag tttttcaaat ggagccctct ttaccagctt gccccctcag 7440

agataagctg ttcccctact tattcagatc tgagatctga aaacattcct tttcctgtga 7500

gttcagctag gacaaagatg gagctttttg ataaaatttg gcaaacacat tttttaaaga 7560

tgaaaatttt taaaaattga aaaaaaaaca tttatagaaa gagacttcta atccaaattt 7620

aacttctcaa actatgtttt gaccggctag cataatgttt cagtctttct ggagaatgcc 7680

ccttgaaact gttttcttct acacaacttc ctcctttcct ttgactttcc tgctctggaa 7740

gggaagaaca ggaagaggac agatcaaatt actcaagagg aaggacaaga aataaggaac 7800

caaattatca acaattggag aaagaaagct gatgtcagta tcatttcata tatgattatg 7860

tcagagtcag gtggataagc caatcctgtt gaatagcata cttttcctgc tactcctgaa 7920

gggtaaagag gtctttctct tacaaagccg tcctagctag taatcttaca ggtgcaaaaa 7980

gcttgttttc atgttatttc ttagtaactc aaaatacctc taaagttata catattatga 8040

aagtactaca gtcacagtgc tgagaaaagg agtaaataag acaatgtata taaaaacact 8100

tggctcagcc cctggctctg tggttgataa atattaagtt agtattcatt attattataa 8160

tttccaaaga gtccattaaa agatatagaa gaagggaggc agcaataaca ctaagagaaa 8220

attccattat ctccaactat ttatcctcta gcccaaaata attgccatta gaaagagcaa 8280

ctttaacaaa aattttaagt tgcaatagat gttcaacttt aaatccatcc cagaaaaatt 8340

tctaaccaaa ggagcataga agatttgatc ttattttcta agtagtatag acttaattgt 8400

gagaacaaaa taaaaacttg 8420

<210> 9
<211> 8475
<212> DNA
<213> Homo sapiens

<400> 9
gcataacttg taagaaatgg agtgaggtct cagttcaaac tggcttctgt atgacttcaa 60

agccaaagtc agcaacttag aaggcaaaaa ttataattta gttggcaaat acgagaaaag 120

gtcagaaaca catgaaatga agctcaatag gaacacttac agggtagcag ggtagtagcc 180

tagggaaaaa agtcagacac taaaattgtt taaataggta agttcaaggg acaggtaaag 240

accttagtgg gtaagaagcc aatcagcaga cgaactgcaa gcaagcactg tctctctttc 300

ccttctgtct cctcttgtag taactgacca caattaaggc tgcctagggg aataatgaag 360

taatcctcct attatcagca atggtctgat ccagtgccag gcaccacaga caacttggtg 420

ttcagagaag atccttcaag atgaacaaag ggtcaaaata aaaaattcta gaagagagaa 480

gactgatcac aatttaatgt aaggcttgga aggaactgat ctctaccttc cttaacatct 540

caagaacttc ctcagattca ttggatgttg agtgtgtgtg agtctagtag aaaaatgaat 600

ttttgtttct taacttggat atgtgattag gatgttaata attaagtctg ggctaatatt 660

gaaggtatct tatgatgggc ttcttaaagc attgatcaca aagactgcat gttcataaac 720

tgagctgcac ttgttaggat tctagatgtt tgaaatttct tgtgttattt tggtctcaga 780

tttctagaca aattttctca aattcctatt tcactttttg acatatcatg agtgactcaa 840

atgtttgccc ttgagtcgga aaacacccag cattaggaat aggcacataa acataatact 900

tcaagcttca gatttaagct caattataaa gtgtttaaag gctgtgctga tagttcttct 960

gagtagaatt cctacaacta tgggtttgtc tataataaaa tgttcactct atattgaacg 1020

ccttatttaa aactcgaaat gtgtaagtag taataaagaa aatatgtcct cctgtaacca 1080

aagctaggac cgattacatg ttcacttgac tgacagatac aatcacctat attaggagca 1140

atcagcactt ccttacaaac taacaacttg agatgtagtg ttcccattgg ctatgaagat 1200

tttctttatt tactcagaat agtctgtagg atctgccagc tgcccctgat tataccagct 1260

gcacccaatg atcacagtga acattatttt acattctaaa taactggtgc aaggtgagcc 1320

atggttttct gagtttccta tcacctttgt gtttcaggtc ctcaaatgtt aatttgtaaa 1380

gctgctgttt caggcaaaac taacaaaatt agcatctaat caataaccat actatgtcca 1440

cccatatcct ataacacaga agtaggggaa gagtgagaaa ggtggaagtg gagaaataga 1500

ggcccaaaaa gaaagtttta tcacaggaat atctagatgt cttctgggat tgtctgttaa 1560

agagctgtga cactcatata aatgcagaat tactctcttt cttccttgtt ggttagaagg 1620

ccaagggtgc catggtaata ctaccaaaca tatatcaaag cttggcagga aaaatggtac 1680

cttcagaaat tttataatct gatatcaaat aggtcaagaa atataataaa actagtttct 1740

ttggtttcct tagaaacctg gaaaacttta aattagaaac ttagaaagct ttaaatcaga 1800

ctttgtagtt aaaaaaggaa attttagttc cttccagcat tagaattccg tgattctctg 1860

actctgagcc tggattaaat ctagcccagc tgagtggaaa cttaagtaac tagctggttg 1920

cctttagtga tcttccactt tatggctgct tccgcctaag aagttcatca tcgtgactta 1980

ctttctttgg ggcaaagtcg tgactaactt tctttggggc aaagttggaa agcagaggtc 2040

aaagtcaatc agaaatggga caaactcact tcctactgcc tggtgaaggg gccattttca 2100

gtagcccctt ttcaagatta gtttcattca agatttgata agctgttttg actttactat 2160

agatcttatt atccatgtca gttaagttta tgcttccact aaatctatct gaattcaaaa 2220

ggtaaaaagc taatgctcag tcttatcaga tttatcttat ttattaatag aatgtggatt 2280

tttttaagca tataacaata atagtaatga taggaccata aatgtggatg gctctttaca 2340

agtcactaac attacataaa ttcctcaaca acacactctg aggccataac aaacttttag 2400

aaataacaca attggctacg gaactccagc catctagctt catgggctcc cactttaatt 2460

tcaaaacaac agaactgtgc acattcattt acatgattag ggcagagctt aactgtatct 2520

catgtagcac ctacatcatt cttcagacaa acttattgcc ttttacagac aagaaaactg 2580

gggctcaaaa aaggacttgc ttataactgg ctaataaaga ggaactctgg gttcaaagtg 2640

agtccaattc tttcttccac ccacagcttc tgctaaagtc attacagaaa tgcatagagc 2700

agttcttcca cgttattgct taggtttcta aagagcagtg acctaataca acatgctcta 2760

taatttatta ctgatttaac tatttcacta aggattcact tttaactttt aacttgtaaa 2820

tatgtctaat aaacaccact gaaatagcaa cctctttctt catggccttg tggttgtaaa 2880

gcaagctagt aatatatgtc tgtggatttg tgctaataaa gttctataca cctcattaat 2940

tccacaaatc ctactgggta tttcttatct gccagatcct acgctaggta ctggatacac 3000

agtactgaac aaaatgggta caaatgagcc tcacagagct tgtttcattg aaaagcagag 3060

agatacacac taatcaacaa attaatagta acacactacg atgtgttttg aaggaaaatt 3120

agagcatcaa agagacggtg ttagcaggtg gaggggagct cttttagatg gagaatgaga 3180

atgcctccct aaagacatgg gaataaattg agatcacaaa aaatgagaaa tagccagcct 3240

tgagaagagc agaaggaaga acattcaaag gaaaagaaag tgcatactgg aaagcctgaa 3300

cactagagtt tggtgtatgt aaggagctga gcaatggtca cttgtgtgat aagatgtgtg 3360

gatgtggggt ggggggcagg ggtgagtccc acgcagctct taagtgtgtc ctcagactcc 3420

tgtggtttcc atcagccaca acctgaataa ctgtgtggta atccaaaaat gattacagat 3480

taaacatata aaaatatcat tacacccata gtacctaagc caaggacaca gtattctatc 3540

ttttcaatga agatctgcat gaagtaaaat tattatatat aattttaggt attgatatag 3600

atacatcagt ggatagatat agatatgtgt ctctggtata gaaaaaagtt ttaaagggat 3660

attaaaagtt cttatcttgc agggttgaag attgtggcaa ctttcatttc tttttaattt 3720

taagaaaaaa gtggtattat gggggattag catgtttgtg ggtatatgta tatttttaat 3780

taaaaaataa acaacaaaat gaaaacgttt ttcttctatg aaagcctaat aagaagaaat 3840

ttcagctgtt ttaacttagg gagctaaaaa catcaaatcc aagaatgttc tctggaactg 3900

agctcaatac atttttattt gagtaagaat tggatacatt tccatcccct tggggctcca 3960

gtctgtcaat attttacttt tcagcgataa aaagacacat gtagataatc acagtgacct 4020

cagtaacttt ccttctctta tttaagttta ttttatttct atcgtagttt tccctgttaa 4080

agattttttc tttttgctta catatataat tttagagaat aacaatgcac acacaaaaaa 4140

ttcctcttgt tctgctagac ctggactttt tctctaatat atatctccat tttttgtctt 4200

ttttcagacg tattttggaa gcaaaggaga gaattgctat atagctgact tcctcttctc 4260

atcaacagtg ttttaacagt ttttaagcaa aagtcagctt tgtttatcta agattttttt 4320

tgctggcatt taacctaccc ctgcctcccc tttcccaagt ccacttcagc caacctctca 4380

ttcgacaggt accaccctct aacataactg aaataatgtc taccattact ggatcttgct 4440

agcaaagaat ctcaaatttt cccacttggt tgtaaattat tttgtaatct ctagtgttta 4500

aggtgcgctt gtcctatcta atcccctccc tggcaggaca ccttacagaa cctacccctt 4560

acactagtca ttaagcacca tcagggacgg atggctgtgt cactggtctg tttggtattc 4620

cctactgatc ctaccatgtg gtgattatct atgacttccc taatccctgg ctgccttagc 4680

tgggactggc tgacatgctt ctcaggttgc cgctggcttt acagtccttt actgcccatg 4740

ccactttgga gataggcagg gctagtactt ttctatataa gcccccaaac ttgactttgt 4800

gtttcacagt aggtgaaaaa gttgggtctc ttttctttta cttttctttc cacaagatga 4860

taaagctagg ggaagcctgt ggacatggtt tatttctgca actgcaatga ttgattggtg 4920

cttcctgctg cttacttcct aaactttgtg ctcagtgtca gatccctagc agtttctatc 4980

ccctgctctg ctaaaaaaga atggatgttg actctcaggc cctagttctt tttaattaaa 5040

ttgtattttt gttatcatta ttattattat tattttgaga tggggtctta ctctgtcgcc 5100

caggctgaag tgcagtggtg caatcacagc tcactgtttt agcctcctga gtagctggga 5160

ctacaagcgt catgccacca tgcttctttt taatttttta aaatggtttt ctgccttcaa 5220

ttctaagcac ttctcaattg taaccaagag ataatacttt ttatgaattc ttaaagttat 5280

caacagatac tcaaagtttt agcaaagtct aaatgatatt aagcttgtcc ttattgccca 5340

agtgacttca atgactattt gttaattgca accaagggtc attttttaaa tgaatatata 5400

ttattattat atatataata ttaaggtcct caaataccta aaagtttagc aaaatctaaa 5460

taatattgtg catattcttt tattactgta ttagtccgtt ttcatgttgc tgataaagac 5520

atacccaaga ctgggcaatt tacaaaagaa agaggttcac tggactcaca gttccacgtg 5580

gctggggagg cctcacaatc acggcagctt acgggattgt tgagaaatga cacttctcaa 5640

gctggggcta aactatctct gtggtagttg ttctgattca agtattgaat tggttttttt 5700

tgtttttttt gagatggagt ttcgttcttg ttgcccaggc tggagtgcaa tggcacgatc 5760

tcagctcacc gcaacctctg cctcccgggt tcaagtgatt ctcctgcttc agcctcccaa 5820

gtagctggga ctacaggcat gagccaccac acccagctaa ttttgtattt ttagtagaga 5880

catggtttct ccatgttggt caggctggtc tcaaactccc aacctcaggt gatccacctg 5940

ccttggcctc ctaaagtgct gggattacag gcataagcca ccgtgcccgg ctggagcatt 6000

ggtatataaa agctgcctag gtaactctaa cctttggccc catacatctg aaggatacct 6060

acaatgcacc tgaaaaatgc aactgaaaca gtagttccct gggaccacac actcagaaag 6120

ggggtgtatc aggagatcta gggaccagga gggtggaaga cctaaggcag cactacagat 6180

gatggagaaa aacccactgg ggaggggcga tcctaacctt gagaatcact gagatcatgc 6240

agaagtattt gatcctacag cattaatatt gtattgtatt gtattagtat atatatatag 6300

tgtatatata tagtattagt atatatattg tattgtatta gcatatatat actaattgta 6360

ttgtattgta tttatatata tagtattgta ttagtatata tatacagtat atatgtatat 6420

atactaatac aatgtactaa tacaatacaa taccatatat atatacacta acacaataca 6480

attagtatat atatatatat atatactaat acaatacaat actatatata tactaataca 6540

atatatacat atatactcac caagacatat tagtggtctg atgtctggct gccacactca 6600

tcttctacct tcagctctgc tctaccaaat atcatttgtt tctgggatct ttgcagtcca 6660

aggaacttca tccttgatat cccacccctt actaactttt tttttttttt ttttttttga 6720

gacggagtct cgctgtgtca cccaggctgg agtgcagtgg tgtgatctcg gctcactgca 6780

agctccacct cctgggatca caccattctc ctgcctcagc ctcccaagta gctgggacta 6840

caggtgcccg ccaccacacc aggctaatgt tttaccgtgt tagcaaggat ggtctcgatc 6900

tcctgacctc atgatccatc cgccttggcc tcctaaagtg ctgggattac aggcataagc 6960

caccgcaccc ggccacccct tactaatttt tagtaacgtc caaggattaa aggaaatttg 7020

ccttacctat ttaacaggaa tcaacagggt taatctcact ccctttctaa aaataattta 7080

taaacattgc agacaatctc atctatccct gtctaaactg tgtggaatta ctgccattta 7140

atgtaatcag tctactcatt tagtttgcct aaggaatttt tgaaaaaaca gttaaatgaa 7200

tgacttaatg gaataaccag gaagttgaag tctccaatag taagaatgaa ctcttgctct 7260

ctggataatc aaatgggtcc ttcctccttc aggtagatca tgccatttcc tcacttacac 7320

tgaacaggta aacaacataa ttactgactt caacttctag ttaattcctt cttttatcac 7380

tgagtatcct ttggctggga gttttgttgg ctatgctgcc atttttctta gttatcacag 7440

tcctataaca taccaatcct tcaatataac tcatctttaa attgtggttt taccttctca 7500

agaagttatt aattatgcca gtgctaaatc ttctaaaatg attgttgact tgttgattag 7560

cccccatgca attcccctct cccgtccctc agcacgtaag gaatggccct ttgcttactt 7620

ccacagatcc ttaaatctac cagttagaag ctaatagcct acctctctac caggaaggaa 7680

ctgtgggctg gaacataata catgttgact tataatttct tagaaaattg tgtgagaaac 7740

atcaaactcc tgattccagg atatgccaaa gacacatcat taaaaagcaa aacaaaacaa 7800

aacaaacctc atttgacgtt gctagtagtg gcatatttca tcaagatcag ctcaaataaa 7860

tagaagtgag attttcacac aaattagact gtagtgcttt tttttttaac ttatctttac 7920

catatgattt ttaacggtaa aaaaaatcgt ttgagatatt agatgtataa tatttatcat 7980

ccaattactt cattagttca atcttttttc aatggcgctc ctgcatctga gaataaggtc 8040

agaaaatttc atgttctgat ttcatgctga ttttcagaag aaaaatgtta gttttgtata 8100

gaataaccca tcctaagaaa tacatttctt attatatttc ttatcttata tttcttagga 8160

caatgagcta ttcaaagggt gatgataacc agcaccatca gtcagcatta tctaagaata 8220

agaatctgtg tttctacata cagacctcct aaaaaggaac ctacacttaa caggattccc 8280

caggcaattt ggatgcacat taaagcttga gcaacactgc attagaaagt tagttttcca 8340

tcacaaaaac agtaacaaaa ggaatataaa gtaagttact ttaataatat aagaagaggg 8400

gcaggccggg cgcagtggct cacgcctgta atcccagcac tttgggaggc tgaggcgggt 8460

ggatcacctg aggtc 8475

<210> 10
<211> 27
<212> DNA
<213> Artificial Sequence

<220>
<223> primer Hspa8-NotI-F

<400> 10
ttcgcggccg ccaaggctga ggcagcg 27

<210> 11
<211> 30
<212> DNA
<213> Artificial Sequence

<220>
<223> primer Hspa8-XbaI-R

<400> 11
ttctctagag gttgctgaaa gaaaaccaaa 30

<210> 12
<211> 34
<212> DNA
<213> Artificial Sequence

<220>
<223> primer Hspa8-NotI-1900F

<400> 12
ttcgcggccg caacaaccta actaatagct gtcc 34

<210> 13
<211> 35
<212> DNA
<213> Artificial Sequence

<220>
<223> primer Hspa8-NotI-1200F

<400> 13
ttcgcggccg caaccttcgc ggccattttg tcctc 35

<210> 14
<211> 33

<212>  DNA
<213>  Artificial Sequence

<220>
<223>  primer Hspa8-human-HindIII-F

<400>  14
ggtgaagctt atacaaacgt tcagaaagtc taa                                    33


<210>  15
<211>  33
<212>  DNA
<213>  Artificial Sequence

<220>
<223>  primer Hspa8-human-EcoT14I-R

<400>  15
ggtgccatgg ggttgctgaa aaaaagaaaa atc                                    33


<210>  16
<211>  33
<212>  DNA
<213>  Artificial Sequence

<220>
<223>  primer Hspa8-human-AatII-F

<400>  16
gggtgacgtc atacaaacgt tcagaaagtc taa                                    33


<210>  17
<211>  35
<212>  DNA
<213>  Artificial Sequence

<220>
<223>  primer Hspa8-mouse-NotI-F

<400>  17
gggtgcggcc gcagaccttc caatttaaac gccac                                  35


<210>  18
<211>  33
<212>  DNA
<213>  Artificial Sequence

<220>
<223>  primer Hspa8-mouse-XbaI-R

<400>  18
gaggtctaga ggttgctatt agaaaaaaaa agg                                    33


<210>  19
<211>  35
<212>  DNA
<213>  Artificial Sequence

<220>
<223>  primer Hspa8-rat-NotI-F

<400>  19
ggtggcggcc gccttttgat agccttcctc acatg                                35


<210>  20
<211>  33
<212>  DNA
<213>  Artificial Sequence

<220>
<223>  primer Hspa8-rat-NheI-R

<400>  20
ggtcgctagc ggttgctaga aggaaaaaaa aaa                                   33